# EUROPEAN PATENT SPECIFICATION

(11) **EP 3 692 959 B1**
(45) Date of publication and mention of the grant of the patent: **27.09.2023**
(21) Application number: 17934675.4
(22) Date of filing: 14.12.2017
(51) Int. Cl.: B29C 65/08, A61F 13/15, B29L 31/48

(54) **ULTRASONIC SEALING METHOD AND ULTRASONIC SEALING DEVICE**
ULTRASCHALLSIEGELVERFAHREN UND ULTRASCHALLSIEGELVORRICHTUNG
PROCÉDÉ DE SCELLEMENT PAR ULTRASONS ET DISPOSITIF DE SCELLEMENT PAR ULTRASONS

(30) Priority: 13.12.2017 JP 2017238626
(43) Date of publication of application: 12.08.2020
(73) Proprietor: Unicharm Corporation, Ehime 799-0111 (JP)
(72) Inventor: NINOMIYA, Akihide, Toyohama-cho, Kanonji-shi, Kagawa 7691602 (JP); YAMAMOTO, Hiroki, Toyohama-cho, Kanonji-shi, Kagawa 7691602 (JP); NAKANO, Takumi, Toyohama-cho, Kanonji-shi, Kagawa 7691602 (JP)
(74) Representative: Staeger & Sperling Partnerschaftsgesellschaft mbB
(86) International application number: PCT/JP2017/044934
(87) International publication number: WO 2019/116508

(56) References cited:
- JP-A- H0 573 155
- JP-A- 2002 355 270
- JP-A- 2002 355 270
- JP-A- 2010 099 132
- JP-A- 2015 212 154

## Description

### [Technical Field]

The present invention relates to a method and a device for ultrasonic-sealing.

### [Background Art]

There is a well-known method for ultrasonic-sealing as follow: a transported continuous web for an absorbent article is clamped between an ultrasonic horn and an anvil, and seal portions are formed in the continuous web by vibration of the ultrasonic horn due to receiving ultrasonic waves generated by an ultrasonic wave generator.

In this method for ultrasonic-sealing, the processing for forming the seal portion is repeatedly executed such that multiple seal portions are formed at intervals in the direction of transporting the continuous web.

Also, in this method for ultrasonic-sealing, a frequency adjusting step is executed in order to adjust the frequency of the ultrasonic waves that the ultrasonic wave generator generates for the ultrasonic horn.

### [Citation List]

### [Patent Literature]

[PTL 1] Japanese Patent Application Publication No. 2002-355270. This prior art defines the preamble of the independent claims.

### [Summary of Invention]

### [Technical Problem]

In conventional examples, the frequency adjusting step is performed when the device for ultrasonic-sealing is powered on (starts up). However, the following problems have occurred in such cases. Specifically, the ultrasonic horn generates frictional heat in the continuous web while performing the seal-portion formation processing, and therefore the ultrasonic horn stretches/contracts due to the thermal influence of the frictional heat. As this seal-portion formation processing continues for an extended period of time, there has been a possibility that the natural frequency of the ultrasonic horn, which changes along with stretching/contracting, will greatly diverge from the ultrasonic frequency that was set by adjustment at power-on (startup).

The present invention was achieved in light of conventional problems such as that described above, and an aspect of the present invention is to suppress the problem that the frequency generated by the ultrasonic wave generator ceases to match the natural frequency of the ultrasonic horn when seal-portion formation processing is executed.

### [Solution to Problem]

Amain aspect of the present invention for achieving the above-described aspect is an method for ultrasonic-sealing in which a seal portion is formed in a continuous web for an absorbent article,
the forming being performed by vibration of the ultrasonic horn due to receiving an ultrasonic wave generated by an ultrasonic wave generator while the continuous web is being transported and clamped between an ultrasonic horn and an anvil,
the method for ultrasonic-sealing including:
   a seal-portion forming step of forming a plurality of the seal portions at an interval in a direction of transporting the continuous web by repeatedly executing processing for forming the seal portion; and
   a frequency adjusting step of adjusting a frequency of the ultrasonic wave generated by the ultrasonic wave generator for the ultrasonic horn and storing the adjusted frequency in a storage unit,
      the adjusting and the storing being performed at a time between
      when the ultrasonic wave generator ends the generation of the ultrasonic wave for the ultrasonic horn in one instance of processing for forming the seal portion and
      when the ultrasonic wave generator starts to generate the ultrasonic wave for the ultrasonic horn in a subsequent instance of processing for forming the seal portion by the ultrasonic horn,
the ultrasonic wave generator starting to generate the ultrasonic wave at the frequency stored in the storage unit when the processing for forming the seal portion is to be executed.

Features of the present invention other than the above will become clear by reading the description of the present specification with reference to the accompanying drawings.

### [Advantageous Effects of Invention]

According to the present invention, it is possible to suppress the problem that the frequency generated by the ultrasonic wave generator ceases to match the natural frequency of the ultrasonic horn when seal-portion formation processing is executed.

### [Brief Description of the Drawings]

FIG. 1 includes an upper diagram that is a perspective view of an unfolded state of a continuous web 30, and a lower diagram that is a perspective view of the continuous web 30 while being transported to a sealing device 1.
FIG. 2 is a cross-sectional view taken along IX-IX in the lower diagram in FIG. 1, and shows a state where the continuous web 30 is clamped between ultrasonic horns 8 and anvils 14.
FIG. 3 is a cross-sectional view taken along X-X in the lower diagram in FIG. 1 and FIG. 2, and shows a state where the continuous web 30 is clamped between ultrasonic horns 8 and anvils 14.
FIG. 4 is a cross-sectional view taken along I-I in FIG. 9, and shows the sealing device 1 according to an embodiment.
FIG. 5 is a diagram showing a rotating drum 5 of the sealing device 1 and the internal structure thereof.
FIG. 6 is an illustrative diagram for illustrating operations of a swing drive portion.
FIG. 7 is a schematic view of a state in which a sealing unit 10 is clamping the continuous web 30.
FIG. 8 is an enlarged view of the internal structure of a holding portion 52.
FIG. 9 is an illustrative view of operation states of the sealing device 1.
FIG. 10 is an illustrative diagram for illustrating operations of six sealing units 10.

### [Description of Embodiments]

At least the following matters will become clear with the description of this specification and the attached drawings.

An method for ultrasonic-sealing in which a seal portion is formed in a continuous web for an absorbent article,
the forming being performed by vibration of the ultrasonic horn due to receiving an ultrasonic wave generated by an ultrasonic wave generator while the continuous web is being transported and clamped between an ultrasonic horn and an anvil,
the method for ultrasonic-sealing including:
   a seal-portion forming step of forming a plurality of the seal portions at an interval in a direction of transporting the continuous web by repeatedly executing processing for forming the seal portion; and
   a frequency adjusting step of adjusting a frequency of the ultrasonic wave generated by the ultrasonic wave generator for the ultrasonic horn and storing the adjusted frequency in a storage unit,
      the adjusting and the storing being performed at a time between
      when the ultrasonic wave generator ends the generation of the ultrasonic wave for the ultrasonic horn in one instance of processing for forming the seal portion and
      when the ultrasonic wave generator starts to generate the ultrasonic wave for the ultrasonic horn in a subsequent instance of processing for forming the seal portion by the ultrasonic horn,
   the ultrasonic wave generator starting to generate the ultrasonic wave at the frequency stored in the storage unit when the processing for forming the seal portion is to be executed.

According to this method for ultrasonic-sealing, the frequency adjusting step is performed at a time between when the ultrasonic wave generator ends the generation of the ultrasonic wave for the ultrasonic horn in one instance of processing for forming the seal portion and when the ultrasonic wave generator starts to generate the ultrasonic wave for the ultrasonic horn in the subsequent instance of processing for forming the seal portion by the ultrasonic horn. This makes it possible to suppress the problem that the frequency generated by the ultrasonic wave generator ceases to match the natural frequency of the ultrasonic horn when the processing for forming the seal portion is executed.

In such a method for ultrasonic-sealing, it is desirable that
when the processing for forming the seal portion is to be executed,
the seal portion is formed while following the frequency of the ultrasonic wave generated by the ultrasonic wave generator, and
the frequency at a time when the ultrasonic wave generator ends the generation of the ultrasonic wave is stored in the storage unit.

According to this method for ultrasonic-sealing, it is possible to further suppress the problem that the frequency generated by the ultrasonic wave generator ceases to match the natural frequency of the ultrasonic horn.

In such a method for ultrasonic-sealing, it is desirable
that in the processing for forming the seal portion,
   the ultrasonic horn and the anvil unclamp the continuous web after the ultrasonic wave generator has stopped generating the ultrasonic wave for the ultrasonic horn,
that in the subsequent processing for forming the seal portion by the ultrasonic horn,
   the ultrasonic wave generator starts to generate the ultrasonic wave for the ultrasonic horn after the ultrasonic horn and the anvil have clamped the continuous web, and
that in the frequency adjusting step,
   the frequency of the ultrasonic wave generated by the ultrasonic wave generator for the ultrasonic horn is adjusted at a time between when the ultrasonic horn and the anvil unclamp the continuous web and when the ultrasonic horn and the anvil subsequently clamp the continuous web.

According to this method for ultrasonic-sealing, the frequency adjusting step is performed at a time between when the ultrasonic horn and the anvil unclamp the continuous web and when the ultrasonic horn and the anvil subsequently clamp the continuous web. This makes it possible to suppress the problem that the frequency generated by the ultrasonic wave generator ceases to match the natural frequency of the ultrasonic horn when the processing for forming the seal portion is executed.

In such a method for ultrasonic-sealing, it is desirable
that after the ultrasonic horn and the anvil unclamp the continuous web,
   the ultrasonic horn moves from a contact state of being in contact with the continuous web to a separated state of being separated from the continuous web,
that after the ultrasonic horn has returned from the separated state to the contact state,
   the ultrasonic horn and the anvil subsequently clamp the continuous web, and
that in the frequency adjusting step,
   the frequency of the ultrasonic wave generated by the ultrasonic wave generator is adjusted at a time between when the ultrasonic horn moves to the separated state and when the ultrasonic horn returns to the contact state.

According to this method for ultrasonic-sealing, frequency adjustment (tuning) can be performed swiftly and accurately.

In such a method for ultrasonic-sealing, it is desirable
that in the frequency adjusting step,
   the ultrasonic wave generator generates the ultrasonic wave, adjusting the frequency of the ultrasonic wave, and
that an amplitude of the ultrasonic wave generated by the ultrasonic wave generator in the frequency adjusting step is smaller than an amplitude of the ultrasonic wave generated by the ultrasonic wave generator in the seal-portion forming step.

According to this method for ultrasonic-sealing, it is possible to suppress the amount of power that is consumed when the ultrasonic wave generator adjusts the frequency.

In such a method for ultrasonic-sealing, it is desirable that
the frequency adjusting step is periodically executed such that the processing for forming the seal portion is performed a predetermined number of times between when the frequency adjusting step is executed and when the frequency adjusting step is subsequently executed.

According to this method for ultrasonic-sealing, the frequency adjusting step can be executed at an appropriate interval regardless of whether the operation mode is a high speed mode or a low speed mode.

In such a method for ultrasonic-sealing, it is desirable
that the continuous web is transported by rotation of the rotation member with being wound on an outer circumferential surface of a rotation member,
that a plurality of sealing units that each have an ultrasonic horn and an anvil are provided at an equal spacing along a circumferential direction of the rotation member,
that a plurality of first members, which are either of the ultrasonic horns and the anvils, are provided on the outer circumferential surface of the rotation member,
that a plurality of second members, which are other ones out of the ultrasonic horns and the anvils, are each provided on an opposite side of the continuous web so as to be capable of moving toward and away from the corresponding first member,
that for each of the sealing units, when the sealing unit reaches a predetermined first angular position while rotating along with rotation of the rotation member,
   the second member comes into contact with the corresponding first member and clamps the continuous web,
that for each of the sealing units, when the sealing unit reaches a predetermined second angular position while rotating along with rotation of the rotation member,
   the second member moves away from the first member and unclamps the continuous web, and
that for each of the sealing units, when the sealing unit reaches a predetermined third angular position along a path from the predetermined second angular position to the predetermined first angular position while rotating along with rotation of the rotation member, the sealing unit execute the frequency adjusting step.

According to this method for ultrasonic-sealing, the frequency adjusting step can be reliably executed at a time between when the ultrasonic wave generator ends the generation of the ultrasonic wave for the ultrasonic horn in one instance of processing for forming the seal portion and when the ultrasonic wave generator starts to generate the ultrasonic wave for the ultrasonic horn in the subsequent instance of processing for forming the seal portion by the ultrasonic horn.

In such a method for ultrasonic-sealing, it is desirable that
for each of the sealing units, the frequency adjusting step is executed each time the predetermined third angular position has been reached by the sealing unit N times (N being a natural number of 2 or more) while rotating along with rotation of the rotation member.

According to this method for ultrasonic-sealing, the ultrasonic frequency can be adjusted at an appropriate timing.

In such a method for ultrasonic-sealing, it is desirable
that the continuous web is transported by rotation of the rotation member with being wound on an outer circumferential surface of a rotation member,
that a plurality of sealing units that each have an ultrasonic horn and an anvil are provided at an equal spacing along a circumferential direction of the rotation member,
that a plurality of first members, which are either of the ultrasonic horns and the anvils, are provided on the outer circumferential surface of the rotation member,
that a plurality of second members, which are other ones out of the ultrasonic horns and the anvils, are each provided on an opposite side of the continuous web so as to be capable of moving toward and away from the corresponding first member,
that the plurality of sealing units sequentially form the plurality of seal portions at the interval while rotating along with rotation of the rotation member, and
that a different ultrasonic wave generator generates the ultrasonic wave for each of the sealing units.

According to this method for ultrasonic-sealing, even if the operation mode is the high speed mode, frequency adjustment can be performed without any problem (with sufficient allowance).

In such a method for ultrasonic-sealing, it is desirable that
when the processing for forming the seal portion is executed,
the ultrasonic wave generator starts to generate the ultrasonic wave, and then the ultrasonic wave generator stops generating the ultrasonic wave when an ultrasonic vibration energy has reached a predetermined amount.

According to this method for ultrasonic-sealing, it is possible to suppress the occurrence of the problem of a sealing defect.

In such a method for ultrasonic-sealing, it is desirable that
in a case where the repeatedly executed processing for forming the seal portion is paused,
the frequency of the ultrasonic wave generated by the ultrasonic wave generator is adjusted when processing is resumed.

According to this method for ultrasonic-sealing, it is possible to further suppress the problem that the frequency generated by the ultrasonic wave generator ceases to match the natural frequency of the ultrasonic horn.

An device for ultrasonic-sealing that forms a plurality of seal portions in a continuous web that is for an absorbent article,
the device for ultrasonic-sealing including:
   a transporting device configured to transport the continuous web;
   an ultrasonic wave generator configured to generate an ultrasonic wave;
   an ultrasonic horn configured to form the seal portion in the continuous web that is being transported by the transporting device, by vibrating due to receiving the ultrasonic wave generated by the ultrasonic wave generator; and
   an anvil configured to clamp the continuous web together with the ultrasonic horn,
the ultrasonic horn forming the plurality of seal portions at an interval in a direction of transporting the continuous web by repeatedly executing ultrasonic-sealing processing,
   the ultrasonic-sealing processing being for forming a seal portion in the continuous web by vibrating while clamping the continuous web together with the anvil,
the ultrasonic wave generator executing a frequency adjusting step of adjusting a frequency of the ultrasonic wave generated by the ultrasonic wave generator for the ultrasonic horn, at a time between
   when the ultrasonic wave generator ends the generation of the ultrasonic wave for the ultrasonic horn in one instance of processing for forming the seal portion and
   when the ultrasonic wave generator starts to generate the ultrasonic wave for the ultrasonic horn in a subsequent instance of processing for forming the seal portion by the ultrasonic horn.

The above device for ultrasonic-sealing achieves operations and effects similar to those of the method for ultrasonic-sealing described above.

### Method for ultrasonic-sealing of present embodiment

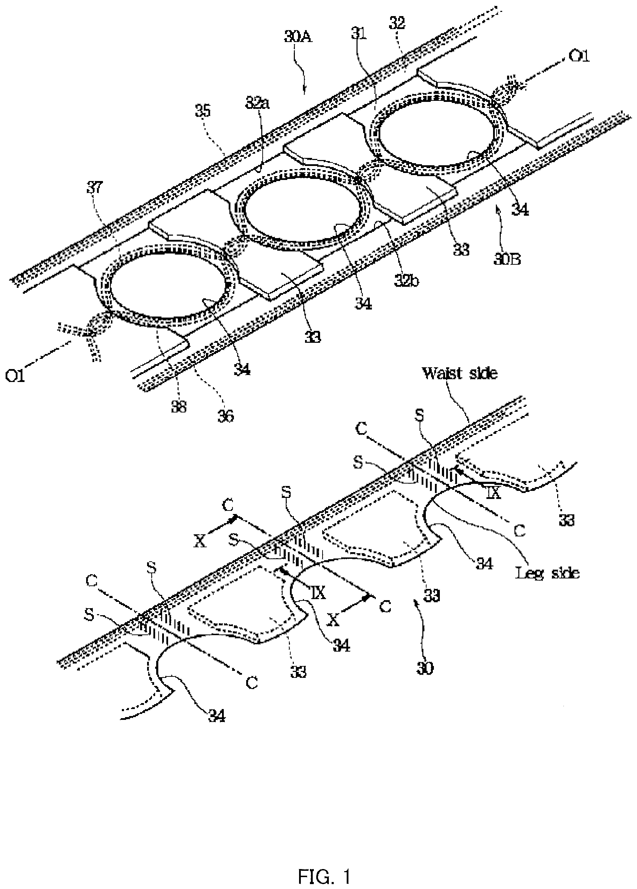

An method for ultrasonic-sealing of the present embodiment is used with an device for ultrasonic-sealing (hereinafter called a sealing device 1) . As shown in the lower diagram in FIG. 1, the sealing device 1 is an device for forming multiple seal portions S in a continuous web 30 at intervals in the direction of transporting the continuous web 30; the continuous web 30 is a stack of absorbent article base sheets and is transported along a continuous production line of the absorbent articles.

### Continuous web 30 for absorbent article

First, the continuous web 30 for an absorbent article of the present embodiment will be described by way of example of a pull-on disposable diaper.

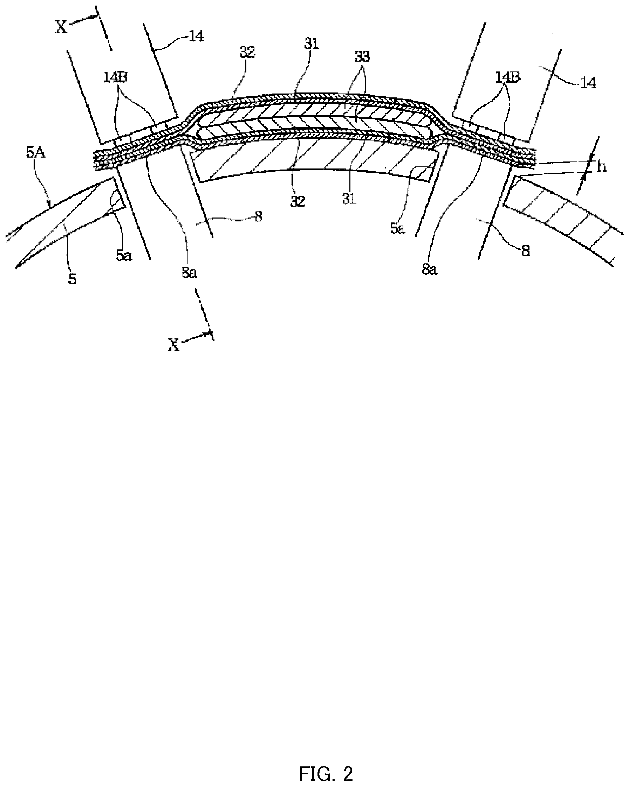

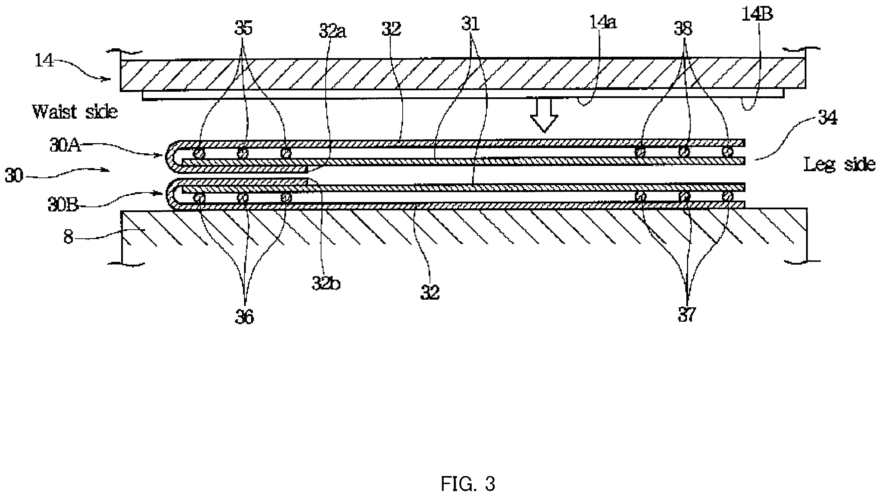

As shown in the lower diagram in FIG. 1, the continuous web 30 of the present embodiment is transported to the sealing device 1 in a folded state. FIG. 2 is cross-sectional view taken along IX-IX in the lower diagram in FIG. 1, and shows a state in which the continuous web 30 is clamped between ultrasonic horns 8 and anvils 14 and seal portions S are being formed in the continuous web 30, and FIG. 3 is a cross-sectional view taken along X-X in the lower diagram in FIG. 1 and FIG. 2.

The lower diagram in FIG. 1 shows a state in which the ultrasonic horns 8 and the anvils 14 of the sealing device 1 have formed seal portions S in the continuous web 30. After the seal portions S have been formed, the continuous web 30 is cut along cut lines C-C between adjacent seal portions S, thus producing pull-on disposable diapers, which are the absorbent article of the present embodiment. Also, the upper diagram in FIG. 1 shows a state in which the continuous web 30 has been unfolded immediately before being transported into the sealing device 1.

As shown in the upper diagram in FIG. 1, when the continuous web 30 is an unfolded belt-like body, a first sheet 32 is located on the underside in the diagram, and a second sheet 31 is stacked thereon. The first sheet 32 is wider than the second sheet 31, and on one side 30A in the upper diagram in FIG. 1, a side edge 32a of the first sheet 32 is folded over onto the second sheet 31. Similarly, on an other side 30B as well, a side edge 32b of the first sheet 32 is folded over onto the second sheet 31. This folded state is shown in FIG. 3.

As indicated by "waist side" in FIG. 3, on the one side 30A of the belt-like body, multiple first waist bands 35 are sandwiched between the first sheet 32 and the second sheet 31. Also, on the other side 30B of the belt-like body, multiple second waist bands 36 are sandwiched between the first sheet 32 and the second sheet 31. The first waist bands 35 and the second waist bands 36 are respectively arranged parallel to each other and extend as straight lines in the direction of transporting the belt-like body.

Also, as indicated by "leg side" in FIG. 3, multiple first leg bands 37 and second leg bands 38 are provided between the first sheet 32 and the second sheet 31. As shown in the upper diagram in FIG. 1, the first leg bands 37 and the second leg bands 38 extend along the direction of transporting the belt-like body while curving in a wave-like shape. Leg holes 34, which form leg insertion portions in the underpants-shaped state, are formed in the regions enclosed within the first leg bands 37 and the second leg bands 38.

The first waist bands 35, the second waist bands 36, the first leg bands 37, and the second leg bands 38 are sandwiched between the first sheet 32 and the second sheet 31 in a state of being stretched by a predetermined factor in the direction of transporting the belt-like body. Also, the first sheet 32 and the second sheet 31 are adhered to the first waist bands 35, the second waist bands 36, the first leg bands 37, and the second leg bands 38, which are sandwiched therebetween, using a hot-melt adhesive or the like.

The first sheet 32 and the second sheet 31 are breathable while also blocking the passage of liquids, and can also be fused together through heat. For example, they can be made of spunbond nonwoven fabric or meltblown nonwoven fabric formed by thermoplastic synthetic fiber, or a laminated body including layers of such nonwoven fabric. Also, a configuration is possible in which either the first sheet 32 or the second sheet 31 is made of nonwoven fabric, and the other one is made of breathable plastic film.

The first waist bands 35, the second waist bands 36, the first leg bands 37, and the second leg bands 38 are elastic stretching members made of rubber or synthetic rubber strings or bands, for example.

A liquid-absorbent body 33 is disposed between adjacent leg holes 34 on the upper surface of the second sheet 31. The liquid-absorbent body 33 is hourglass-shaped or rectangular, and multiple liquid-absorbent bodies 33 are arranged at constant intervals in the direction of transporting the belt-like body. The liquid-absorbent body 33 includes a pulverized pulp, a mixture of a pulverized pulp and a liquid-absorbent polymer (SAP), a stacked body including layers of hydrophilic nonwoven fabric, an air-laid pulp, or the like. These absorbent materials are wrapped in a liquid-permeable top sheet. Each liquid-absorbent body 33 is adhered to the upper surface of the second sheet 31 using a hot-melt adhesive or the like.

The top sheet is formed by spun-lace nonwoven fabric, air-through nonwoven fabric, a plastic film provided with liquid passage holes, or the like.

The belt-like body shown in the upper diagram in FIG. 1 is folded one time onto itself at a center line O1-O1 that extends in the longitudinal direction, thus obtaining the continuous web 30 shown in the lower diagram in FIG. 1. As previously described, the continuous web 30 is transported to the sealing device 1 in the form shown in the lower diagram in FIG. 1. Upon the continuous web 30 being transported to the sealing device 1, the first sheet 32 and the second sheet 31, which are overlaid on each other, are clamped and sealed between the ultrasonic horns 8 and the anvils 14 of the sealing device 1 at locations between adjacent liquid-absorbent bodies 33.

Also, in the present embodiment, as shown in FIG. 3, in intermediate portions of the continuous web 30 that are sandwiched between the ultrasonic horns 8 and the anvils 14, four sheets altogether, specifically two portions of the first sheet 32 and two portions of the second sheet 31, are overlaid on each other. The thickness of the continuous web 30 is the smallest in the intermediate portions. On the waist side, in addition to the four sheets, the two side edges of the first sheet 32 are folded back, and the first waist bands 35 and the second waist bands 36 are also sandwiched between the sheets, thus forming the thickest portions. On the leg side corresponding to the leg hole 34 side, the four sheets and the first leg bands 37 and the second leg bands 38 arranged therebetween are provided, and the thickness on the leg side is higher than in the intermediate portions, but lower than on the waist side.

The first sheet 32 and the second sheet 31 are formed from a heat-fusing material, and therefore when heat is generated therein by vibration applied by the ultrasonic horn 8, the sheets become fused together in accordance with a micro protrusion pattern that is formed on an anvil facing surface 14a of the anvil 14, thus forming seal portions S.

In the example shown in the lower diagram in FIG. 1, the pattern of the seal portions S formed by the micro protrusion pattern is a column of thin seal lines. After the seal portions S have been formed by the sealing device 1, the continuous web 30 is cut along cut lines C-C located between adjacent seal portions S, thus forming pull-on disposable diapers corresponding to the absorbent article.

Note that although the manufacturing of a pull-on disposable diaper has been described above as an example of the manufacturing of an absorbent article, the absorbent article manufactured by the sealing device 1 of the present invention may be a sanitary napkin, a panty liner, or the like.

### Sealing device 1

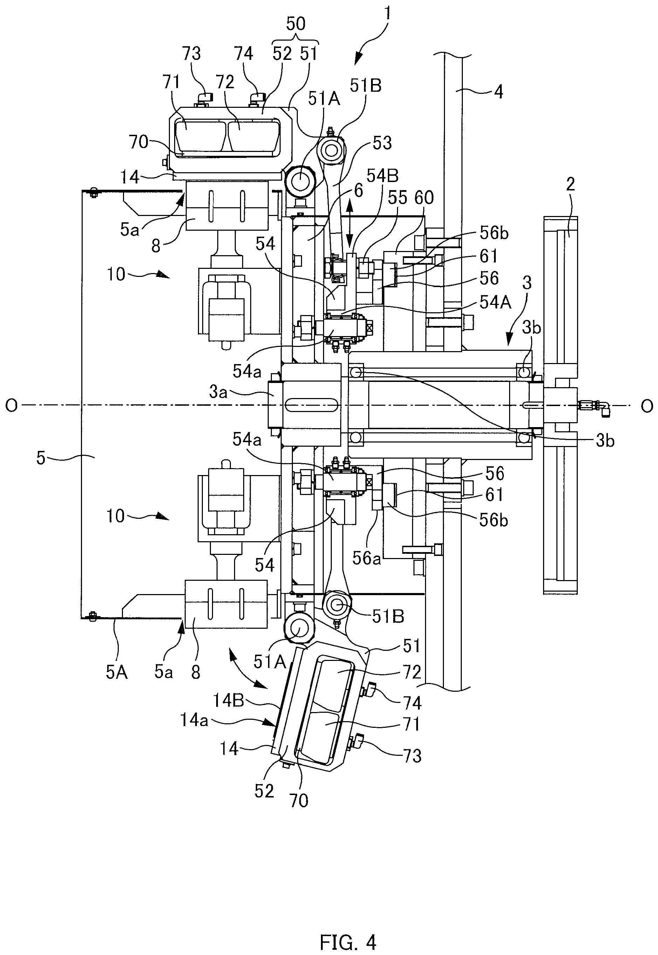

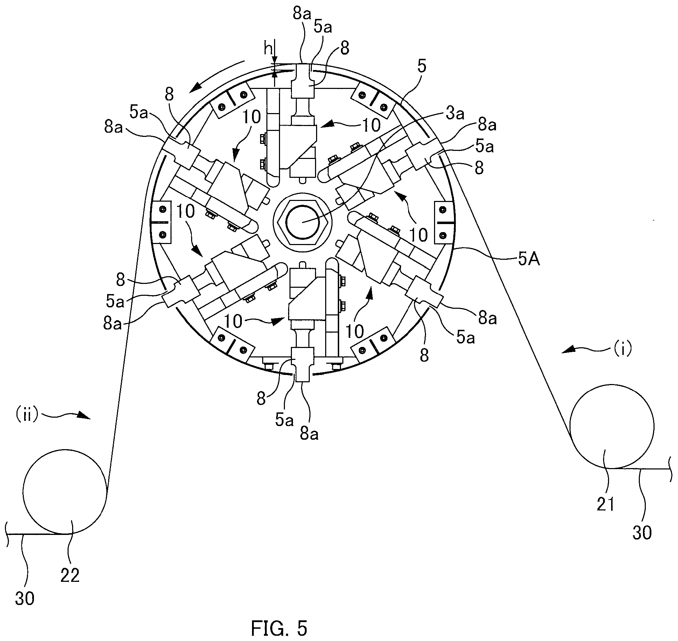

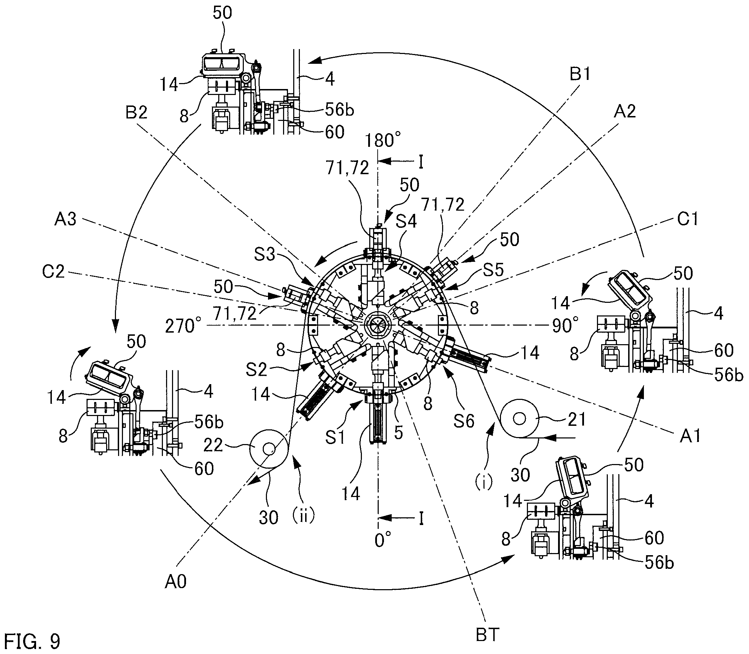

Next, the sealing device 1 will be described. FIG. 4 is a cross-sectional view of the sealing device 1 according to the present embodiment (a cross-section along line I-I in FIG. 9) . FIG. 5 is a diagram showing a rotating drum 5 of the sealing device 1 and the internal structure thereof.

The sealing device 1 includes a rotation-member drive portion, a rotation member (corresponding to a transporting device), sealing units 10, ultrasonic wave generators 9, swinging support members 50, and a swing drive portion.

As shown in FIG. 4, the rotation-member drive portion includes a rotation shaft 3a, a bearing portion 3, ball bearings 3b, a timing wheel 2, a toothed belt (not shown), and a motor (not shown). Also, the bearing portion 3 is provided on a fixed table 4, which is a fixed portion, and the rotation shaft 3a is rotatably supported by the ball bearings 3b that are held in the bearing portion 3. In FIG. 4, the center line of the rotation shaft 3a is indicated by a rotation center line O-O.

The timing wheel 2, which has teeth on its outer circumferential surface, is fixed to the base end portion of the rotation shaft 3a on the right side in FIG. 4, and the toothed belt is hung around the timing wheel 2. When a drive source that has a motor applies motive power to the timing wheel 2 via the toothed belt, the rotation shaft 3a continuously rotates in the counter-clockwise direction as seen from the left side in FIG. 4.

The rotation member is for transporting the continuous web 30, and includes a rotation base 6 and the rotating drum 5 as shown in FIG. 4. The rotation member is fixed to the rotation shaft 3a, and rotates due to the rotation-member drive portion. Specifically, the rotation base 6 is fixed to the rotation shaft 3a, the rotating drum 5 is fixed to the rotation base 6, and these members rotate along with the rotation shaft 3a. The rotation base 6 is parallel with and opposes the fixed table 4 across a gap for ensuring space for provision of the swinging support members 50 and the swing drive portion.

As shown in FIGS. 4 and 5, an outer circumferential surface 5A of the rotating drum 5 is provided with multiple windows 5a that are rectangular and extend parallel with the rotation center line O-O. The windows 5a are formed at an even pitch in the circumferential direction of the rotating drum 5, and in the present embodiment, six windows 5a are provided at an arrangement angle of 60 degrees about the rotation center line O-O.

The sealing units 10 each include an ultrasonic horn 8, a booster 7b, a converter 7a, and an anvil 14, and are provided on the rotation member.

Accordingly, the sealing units 10 rotate along with the rotation base 6 and the rotating drum 5. In the present embodiment, as shown in FIG. 5, multiple (six) sealing units 10 are provided at an equiangular arrangement angle of 60 degrees along the circumferential direction of the rotation member. Hereinafter, the sealing units 10 will be called a sealing unit S1, a sealing unit S2, a sealing unit S3, a sealing unit S4, a sealing unit S5, and a sealing unit S6, in order along the circumferential direction.

As shown in FIG. 4, the ultrasonic horn 8 is fixed to the rotation base 6 at a location inside the rotating drum 5. The ultrasonic horns 8 and the devices connected to the ultrasonic horns 8 are arranged in a radiating manner about the rotation center line O-O, and the arrangement angle thereof matches the arrangement angle of the windows 5a. Also, as shown in FIG. 5, the ultrasonic horns 8 each protrude outward from the corresponding window 5a of the rotating drum 5 to a projecting height h, a horn facing surface 8a at the leading end of the ultrasonic horn 8 faces outward in the normal direction (radial direction) from the rotation center line O-O, and the horn facing surface 8a is parallel with the rotation center line O-O.

The anvils 14 are fixed to support portions 70 of holding portions 52 of the swinging support members 50, and move toward and away from the horn facing surfaces 8a as the swinging support members 50 swing. In other words, the anvils 14 each move to a position in contact with the corresponding ultrasonic horn 8, and move to a distant position rotated about 90 degrees.

Note that the sealing units 10 will be described in further detail later.

As shown in FIG. 4, each of the swinging support members 50 is constituted by a swing portion 51 and a holding portion 52, and the swing portion 51 and the holding portion 52 are fixed. The anvils 14 are fixed to the support portions 70, which are provided on the holding portions 52. The swinging support members 50 are provided on the rotation member. Accordingly, the swinging support members 50 rotate along with the rotation base 6 and the rotating drum 5. Note that the holding portions 52 will be described in further detail later.

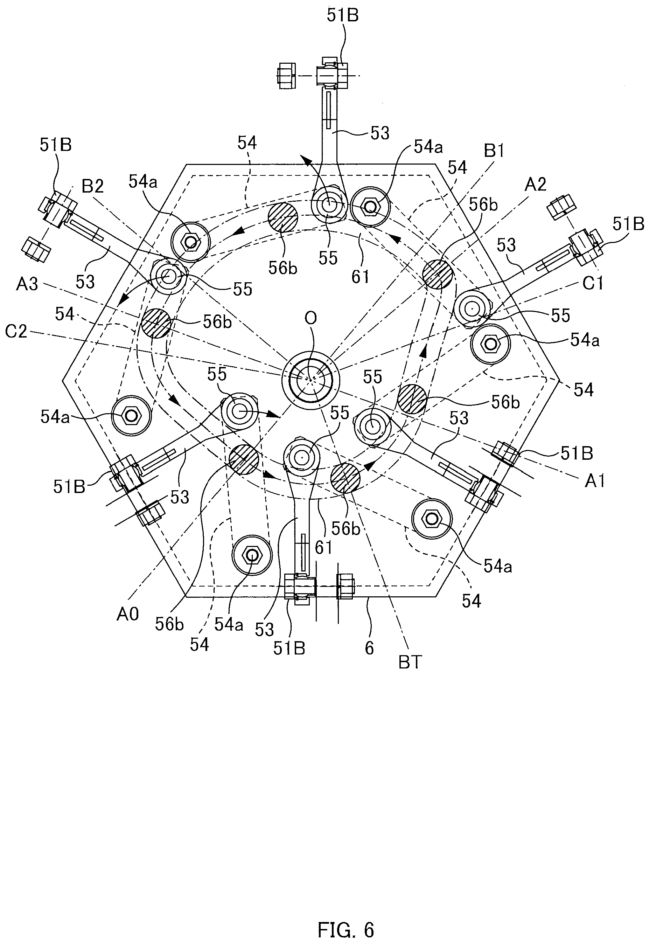

FIG. 6 is an illustrative diagram for illustrating swinging operations of the swinging support members 50. As shown in FIG. 6, the rotation base 6 is has a regular hexagonal front surface. The six swinging support members 50 are provided at intervals of 60 degrees at the center portions of the sides of the regular hexagon, and the arrangement angle of the swinging support members 50 matches the arrangement angle of the ultrasonic horns 8.

As shown in FIG. 4, the swing portions 51 are swingably supported to respective sides of the regular hexagon of the rotation base 6 by swing shafts 51A, and the swing shafts 51A are oriented orthogonal to the rotation center line O-O. Accordingly, the swinging support members 50 (holding portions 52) can swing the anvils 14 about the swing shafts 51A to a position radiating away from rotation center line O-O.

As shown in FIG. 4, in the swing drive portion, a cam member 60 that constitutes the swing drive portion is fixed to the upper surface of the fixed table 4 that faces the rotation base 6. The cam member 60 is shaped as a flat plate that has a predetermined thickness, and as shown in FIG. 6, the front surface is provided with a cam groove 61 that serves as a cam track. The cam groove 61 is continuous around the rotation center line O-O. Also, the recession depth direction of the cam groove 61 is parallel with the rotation center line O-O.

As shown in FIGS. 4 and 6, a link mechanism is provided between the cam member 60 and each of the swing portions 51. In the link mechanism, drive links 53 are rotatably supported to the swing portions 51 via first coupling shafts 51B that are oriented orthogonal to the rotation center line O-O.

Base portions 54A of rotation links 54 are rotatably supported to the rear surface of the rotation base 6 by support shafts 54a. The axial directions of the support shafts 54a are parallel with the rotation center line O-O. The leading end portions of the drive links 53 are rotatably coupled to the leading end portions of arm portions 54B of the rotation links 54 via second coupling shafts 55. The axial directions of the second coupling shafts 55 are parallel with the rotation center line O-O.

A drive member 56 is attached to each of the rotation links 54. The drive member 56 has a drive support body 56a that is non-rotatably fixed to an intermediate portion of the rotation link 54, and a follower 56b that is provided on the drive support body 56a. The follower 56b can move inside the cam groove 61. The follower 56b rolls inside the cam groove 61, or slides therein without rolling.

Note that FIG. 6 shows the relative positional relationship that the rotation links 54 and the followers 56b have with the drive links 53, and the drive support bodies 56a are not shown.

As shown in FIG. 6, when the rotation base 6 rotates, the followers 56b move along the cam groove 61. At this time, the distance between each of the followers 56b and the rotation center line O-O changes according to the shape of the cam groove 61. The rotation links 54 rotate in accordance with this change, and the swinging support members 50 also rotate via the drive links 53.

As previously described, the swinging support members 50 rotate due to the cam groove 61, and as a sealing unit 10 moves from an angular position A2 to an angular position A3 shown in FIG. 6, the swinging support member 50 rotates toward the outer circumferential surface 5A of the rotating drum 5 and then reaches a contact position, as shown by the sealing unit 10 at the top in FIG. 4. Also, as the sealing unit 10 moves from an angular position A0 to an angular position A1 shown in FIG. 6, the swinging support member 50 rotates by about 90 degrees toward the fixed table 4 to a retracted position (see FIG. 9), as shown by the sealing unit 10 at the bottom in FIG. 4.

### Sealing units 10 and ultrasonic wave generators 9

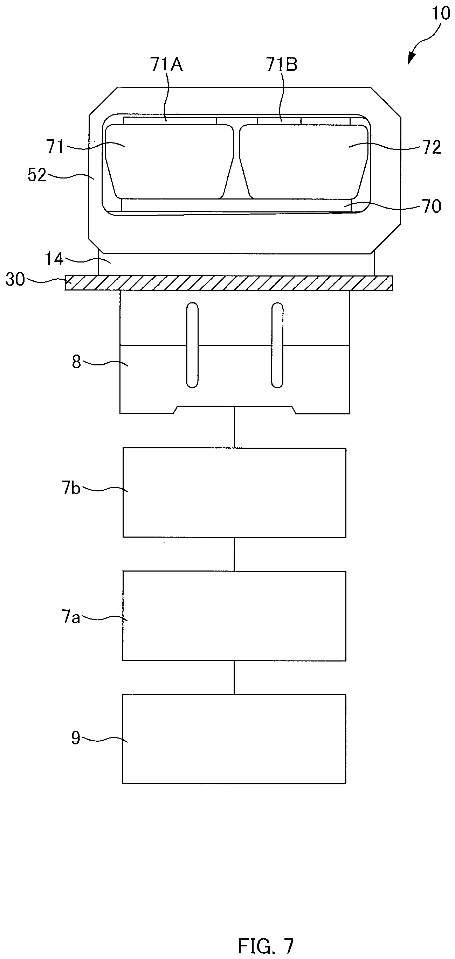

FIG. 7 is a schematic view of a state in which one of the sealing units 10 is clamping the continuous web 30.

In the sealing unit 10, the transported continuous web 30 is clamped between the ultrasonic horn 8 and the anvil 14, an ultrasonic signal emitted (generated) by the ultrasonic wave generator 9 is converted into mechanical ultrasonic vibration by the converter 7a, and the ultrasonic horn 8 receives the mechanical ultrasonic vibration via the booster 7b. The ultrasonic horn 8 vibrates upon receiving the mechanical ultrasonic vibration, and a seal portion S is thus formed in the continuous web 30.

In the present embodiment, a different ultrasonic wave generator 9 generates ultrasonic waves for each of the sealing units 10. In other words, one ultrasonic wave generator 9 is provided for each of the sealing units 10. Accordingly, as shown in FIG. 7, each sealing unit 10 has one ultrasonic horn 8, one booster 7b, one converter 7a, and one anvil 14, and is connected to one ultrasonic wave generator 9. Note that the ultrasonic wave generators 9 are provided outside of the rotation member and are connected thereto via a slip ring (not shown) . Also, first members, which are either the ultrasonic horns 8 or the anvils 14 (in the present embodiment, the ultrasonic horns 8), are provided on the outer circumferential surface 5A of the rotation member (rotating drum 5), and second members, which are the other ones (in the present embodiment, the anvils 14), are provided on the opposite side of the continuous web 30 so as to be able to move toward and away from the first members (ultrasonic horns 8).

The ultrasonic wave generator 9 is a device that emits ultrasonic waves (an ultrasound electrical signal) . The frequency of the ultrasonic waves emitted by the ultrasonic wave generator 9 (hereinafter, also called the ultrasonic frequency) is equivalent to the natural frequency of the ultrasonic horn 8 (i.e., the resonance frequency, which in stricter terms is the natural frequency of the ultrasonic stack including the converter 7a and the booster 7b as well, but will be described here as the natural frequency of the ultrasonic horn 8). In other words, the size (length) of the ultrasonic horn 8 is designed such that the ultrasonic frequency emitted by the ultrasonic wave generator 9 (in the present embodiment, 20kHz) is equivalent to the natural frequency of the ultrasonic horn 8.

The converter 7a is connected to the ultrasonic wave generator 9, and converts the ultrasound electrical signal emitted by the ultrasonic wave generator 9 into mechanical vibration. The converter 7a is provided with a piezoelectric element, and the aforementioned conversion is performed by the piezoelectric element.

The booster 7b is connected to the converter 7a, and amplifies the vibration received from the converter 7a.

The ultrasonic horn 8 is connected to the booster 7b. And, the ultrasonic horn 8 generates frictional heat at the interface between overlaid base materials in the continuous web 30 by vibrating due to receiving ultrasonic waves emitted by the ultrasonic wave generator 9 (ultrasonic waves in the form of mechanical vibration due to the conversion into mechanical vibration performed by the converter 7a), that is to say by applying mechanical energy from vibration to the continuous web 30. Accordingly, the base materials melt and form a seal portion S in the continuous web 30.

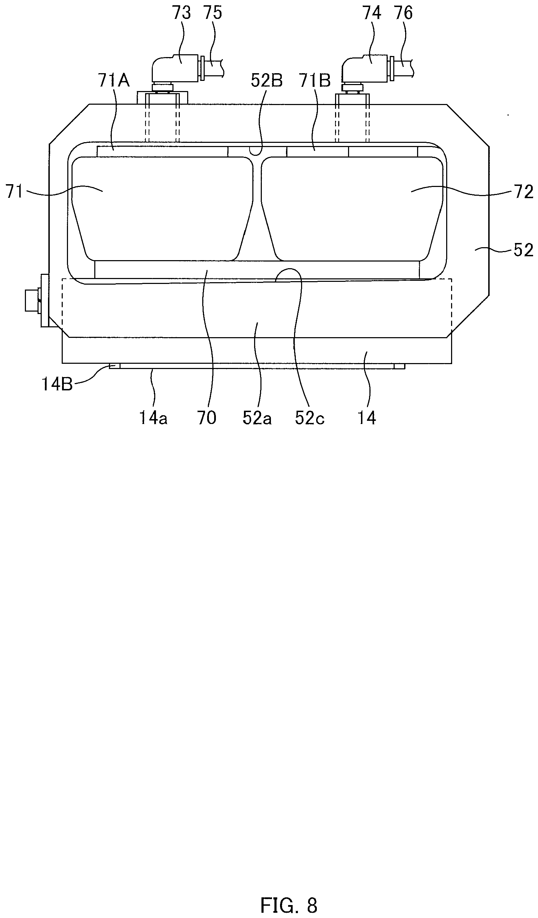

The anvil 14 is a member for performing sealing by clamping the continuous web 30 together with the ultrasonic horn 8. The surface of the anvil 14 is provided with projection portions 14B (see FIG. 8), and the surfaces of the projection portions 14b make up the anvil facing surface 14a that faces the ultrasonic horn 8. The anvil facing surface 14a is a patterned surface set with a pattern corresponding to the seal that is to be formed in the continuous web 30. The anvil 14 is fixed to a support portion 70, which is provided inside the holding portion 52, so that the patterned surface faces outward from the holding portion 52, and the support portion 70 supports a first elastic member 71 and a second elastic member 72 that will be described later.

### Frequency adjusting (tuning) function of ultrasonic wave generator 9

The size (length) of the ultrasonic horn 8 can change due to wear over time and/or expansion or contraction in temperature. If the length of the ultrasonic horn 8 changes, its natural frequency will also change. If the natural frequency changes, a problem occurs in which the ultrasonic frequency emitted by the ultrasonic wave generator 9 no longer matches the natural frequency, and this can cause a problem such as a seal failure in the continuous web 30 (The details will be described later).

In order to suppress the occurrence of such problems, the ultrasonic wave generator 9 has, as a standard function, a frequency adjusting (tuning) function for adjusting the frequency of the ultrasonic waves that the ultrasonic wave generator 9 applies to the ultrasonic horn 8. When frequency adjustment is performed, the ultrasonic wave generator 9 actually emits ultrasonic waves for frequency adjustment, and detects the ultrasonic frequency that corresponds to the natural frequency of the ultrasonic horn 8. The detected ultrasonic frequency is used when seal-portion formation processing is subsequently performed by the ultrasonic horn 8.

In the present embodiment, the adjusted (detected) frequency is temporarily stored in a storage unit such as a memory provided in the ultrasonic wave generator 9. When seal portion formation is to be subsequently performed, that frequency is read out from the storage unit, the ultrasonic wave generator 9 starts to emit ultrasonic waves in accordance with the ultrasonic frequency.

Note that the ultrasonic wave generator 9 of the present embodiment has, as a standard function and as a different function from the tuning function, a function that is for, when executing seal-portion formation processing, forming a seal portion S while adjusting the ultrasonic frequency in accordance with change thereof, the change being caused by vibration with the continuous web 30 being clamped by the ultrasonic horn 8 and the anvil 14. In other words, even when seal-portion formation processing is performed, the natural frequency can change from time to time, and the ultrasonic wave generator 9 changes the ultrasonic frequency while tracking such change in the natural frequency. Accordingly, in the present embodiment, the ultrasonic frequency acquired by the frequency adjusting function is only used when starting to emit ultrasonic waves.

Also, in the present embodiment, the amplitude of the ultrasonic waves emitted by the ultrasonic wave generator 9 in frequency adjustment is smaller than the amplitude of the ultrasonic waves emitted by the ultrasonic wave generator 9 when forming the seal portion S. The ultrasonic waves emitted by the ultrasonic wave generator 9 in frequency adjustment do not need an amplitude sufficient for melting the continuous web 30, and it is sufficient to be able to detect the ultrasonic frequency that corresponds to the natural frequency of the ultrasonic horn 8. Accordingly, in the present embodiment, the amplitude of the ultrasonic waves emitted by the ultrasonic wave generator 9 in frequency adjustment is about 5% of the amplitude of the ultrasonic waves emitted by the ultrasonic wave generator 9 when forming the seal portion S. This therefore enables suppressing the amount of power that is consumed when the ultrasonic wave generator 9 performs frequency adjustment.

### Holding portion 52

FIG. 8 is an enlarged view of the internal structure of one holding portion 52. The holding portion 52 includes the first elastic member 71 and the second elastic member 72, and the anvil 14 is fixed to the support portion 70 that supports the first elastic member 71 and the second elastic member 72. The first elastic member 71 and the second elastic member 72 are arranged side-by-side in the width direction of the rotating drum 5, that is to say in a direction along the rotation center line O-O. The holding portion 52 swings due to the swing drive portion so as to move the anvil 14 toward or away from the ultrasonic horn 8. The anvil 14 and the ultrasonic horn 8 clamp the continuous web 30 when the holding portion 52 is at a contact position at which the anvil 14 and the ultrasonic horn 8 are in contact with each other.

The first elastic member 71 and the second elastic member 72 are each an air damper or an air spring that includes a bag body (casing) that is flexibly deformable and elastically deformable and made of an elastically deformable material such as rubber or rubber embedded with a reinforcing material. The hollow portion of the bag body receives a supply of air as a fluid such that the interior is set to a predetermined pressure.

As shown in FIG. 8, the attachment sides of the first elastic member 71 and the second elastic member 72 are fixed to an attachment surface 52B of the holding portion 52 via a first fixing plate 71A and a second fixing plate 71B. The support portion 70 is fixed to the pressure application sides of the first elastic member 71 and the second elastic member 72, and the anvil 14 is fixed to the support portion 70. When the anvil 14 is not in contact with the ultrasonic horn 8, the support portion 70 is pressed against an inward surface 52c of a frame portion 52a due to the fluid pressure in the first elastic member 71 and the second elastic member 72.

The first fixing plate 71A and the second fixing plate 71B are respectively provided with a first nozzle 73 and a second nozzle 74, and the first nozzle 73 and the second nozzle 74 are respectively connected to a first air pipe 75 and a second air pipe 76. In other words, the first elastic member 71 and the second elastic member 72 each have an air supply portion, and the internal pressures thereof can be set individually.

As previously described, the continuous web 30 of the present embodiment is the above-described continuous body of pull-on disposable diapers, and as shown in FIG. 3, the continuous web 30 is not flat in the portions where the seal portions S are formed by the ultrasonic horns 8 and the anvils 14. In other words, the thickness is highest on the waist side, the thickness is lower in the intermediate portion, and the thickness is then higher on the leg side. Accordingly, the continuous web 30 that is to be subjected to sealing has different thicknesses in different portions, and the surface has an uneven shape.

Even in the case where the thickness is not even at the locations where the seal portions S are to be formed, and instead is different at different locations as with a disposable diaper, the anvil facing surface 14a can flexibly accommodate changes in the thickness of the continuous web 30 because the anvil 14 is pressed against the ultrasonic horn 8 by the air dampers that are bag-shaped elastic bodies filled with air (a fluid). In other words, due to the pressure inside the first elastic member 71 and the second elastic member 72, the ultrasonic horn 8 and the anvil 14 can substantially evenly press the locations where the seal portions S are to be formed in the continuous web 30. Accordingly, the seal quality can be made uniform for the seal portion S locations.

Also, the pressure inside the bag bodies of the first elastic member 71 and the second elastic member 72 can be controlled by the supply of air from the first nozzle 73 and the second nozzle 74. Accordingly, control for changing the pressure inside the bag bodies can be easily performed in accordance with the material and the structure of the continuous web 30 that is to be subjected to sealing. For this reason, even if the structure of the continuous web 30 is changed, and the sealing pattern of the anvil facing surface 14a is accordingly changed, simply changing the pressure inside the bag bodies makes it possible to complete the setting for that stage, and to always perform sealing with optimal conditions.

### Operations of sealing device 1

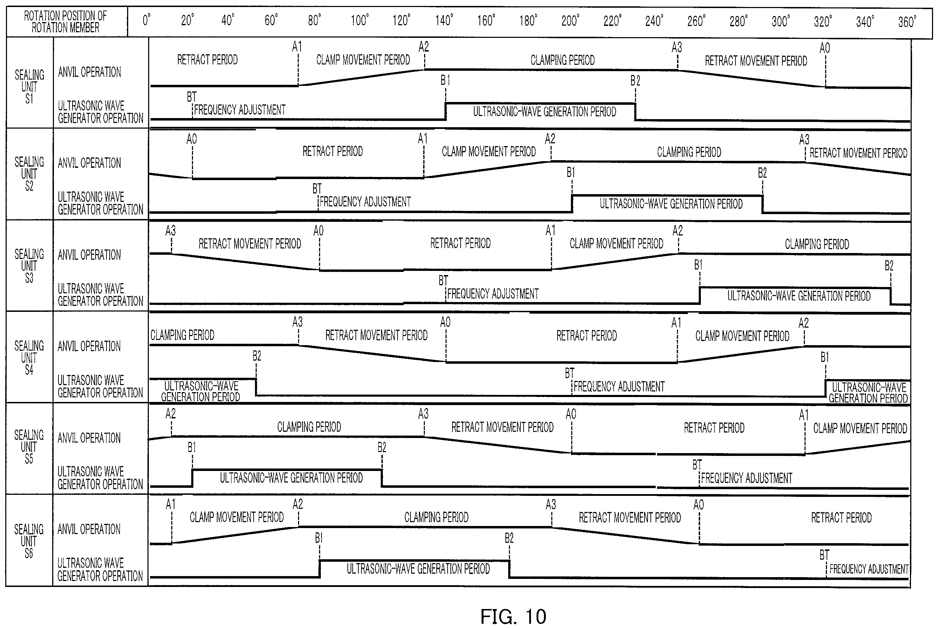

Next, operations of the sealing device 1 will be described with reference to FIGS. 1 to 6 and 9 to 10. FIG. 9 is an illustrative view of operation states of the sealing device 1. FIG. 10 will be described later.

As shown in FIG. 5, the continuous web 30 is wound on a supply transporting roll 21 and transported to the outer circumferential surface 5A of the rotating drum 5. The continuous web 30 is wound on the outer circumferential surface 5A of the rotating drum 5 (more specifically, the horn facing surfaces 8a of the ultrasonic horns 8 that project from the outer circumferential surface 5A) over an angle of about 150 degrees, and then separates from the rotating drum 5 in a discharge section (ii), is wound around a discharge transporting roll 22, and is drawn to the outside.

The continuous web 30 is continuously fed to the supply section (i) at a constant speed, and in the sealing device 1, rotation motive power is transmitted to the timing wheel 2, and the rotation shaft 3a as well as the rotating drum 5 and the rotation base 6 (which constitute the rotation member) rotate at a constant angular speed in the counter-clockwise direction in FIGS. 5 and 9.

Here, the continuous web 30 comes into contact with the horn facing surfaces 8a of the ultrasonic horns 8 that project from the outer circumferential surface 5A of the rotating drum 5, and the rotating drum 5 rotates in this state. In the present embodiment, the angular speed of the rotation member is set such that the rotation circumferential speed of the horn facing surfaces 8a matches the supply speed of the continuous web 30. Accordingly, on the outer circumferential surface 5A of the rotating drum 5, the horn facing surfaces 8a of the ultrasonic horns 8 and the continuous web 30 move together without sliding relative to each other. In this way, the continuous web 30 is transported due to rotation of the rotation member (rotating drum 5) while the continuous web 30 is wound on the outer circumferential surface 5A of the rotation member (rotating drum 5).

Also, the circumferential arrangement pitch of the ultrasonic horns 8 that project from the outer circumferential surface 5A of the rotating drum 5 matches the arrangement pitch of the liquid-absorbent bodies 33 and the arrangement pitch of the leg holes 34 in the continuous web 30 shown in the lower diagram in FIG. 1. Accordingly, when the continuous web 30 is transported to the outer circumferential surface 5A of the rotating drum 5, as shown in FIG. 2, each liquid-absorbent body 33 is located between a pair of adjacent ultrasonic horns 8, and portions not including the liquid-absorbent body 33 are arranged on the horn facing surfaces 8a of the ultrasonic horns 8.

While the rotation base 6 and the rotating drum 5 rotate in the counter-clockwise direction, the followers 56b provided in the swing drive portion move along the cam groove 61 of the cam member 60 provided on the fixed table 4.

As shown in FIGS. 6 and 9, as each sealing unit 10 (the anvil 14 and the ultrasonic horn 8) moves from the predetermined angular position A0 to the predetermined angular position A1 due to the rotation of the rotation base 6, the corresponding follower 56b moves toward the rotation center line O-O due to the cam groove 61. Accordingly, at this time, as shown by the sealing unit 10 at the bottom in FIG. 4, the corresponding swinging support member 50 rotates about the swing shaft 51A so as to face outward in the radiating direction, and the anvil 14 held by the swinging support member 50 faces outward at an angle of about 90 degrees relative to the rotation center line O-O.

When the sealing unit 10 (the anvil 14 and the ultrasonic horn 8) moves from the angular position A1 to the predetermined angular position A2 (corresponding to a predetermined first angular position), the follower 56b is guided by the cam groove 61 so as to move circumferentially outward. Accordingly, the swinging support member 50 rotates toward the outer circumferential surface 5A of the rotating drum 5, and when the angular position A2 is reached, the anvil 14 and the ultrasonic horn 8 clamp the continuous web 30 (the anvil 14 comes into contact with the ultrasonic horn 8, and clamps the continuous web 30) . Specifically, as shown in FIG. 2, in the region of the continuous web 30 in which the liquid-absorbent body 33 is not provided, the stacked body, which includes the first sheet 32, the second sheet 31, the first waist bands 35, the second waist bands 36, the first leg bands 37, and the second leg bands 38, is compressed between the horn facing surface 8a of the ultrasonic horn 8 and the anvil facing surface 14a of the anvil 14.

Note that as shown in FIG. 9, when the sealing unit 10 (the anvil 14 and the ultrasonic horn 8) reaches a predetermined angular position C1 that is along the path from the angular position A1 to the angular position A2, the ultrasonic horn 8 moves from a separated state of being separated from the continuous web 30 to a contact state of being in contact with the continuous web 30.

This clamping state continues until the predetermined angular position A3 (corresponding to a predetermined second angular position) is reached, and during that time (i.e., while the continuous web 30 is clamped by the anvil 14 and the ultrasonic horn 8 from the angular position A2 to the angular position A3), the seal portions S are formed. In other words, the processing for forming the seal portions S is processing in which the ultrasonic horn 8 and the anvil 14 clamp the continuous web 30, the seal portions S are formed in the continuous web 30 by vibration of the ultrasonic horn 8 due to receiving ultrasonic waves emitted by the ultrasonic wave generator 9, and then the continuous web 30 is unclamped. This ultrasonic-sealing processing is executed in the period of time from the angular position A2 to the angular position A3.

When the sealing unit 10 (the anvil 14 and the ultrasonic horn 8) reaches a predetermined angular position B1, the ultrasonic wave generator 9 starts to emit ultrasonic waves for the ultrasonic horn 8. Note that in the present embodiment, the angular position of the sealing unit 10 can be detected by an encoder, and an electrical signal is used to notify the ultrasonic wave generator 9 that the sealing unit 10 (the anvil 14 and the ultrasonic horn 8) has reached the angular position B1. The ultrasonic horn 8 vibrates upon receiving ultrasonic waves from the ultrasonic wave generator 9, and forms a seal portion S in the continuous web 30.

After the ultrasonic wave generator 9 starts to emit ultrasonic waves, when the ultrasonic vibration energy (which corresponds to the electric energy of the ultrasonic wave generator 9) reaches a predetermined amount, the ultrasonic wave generator 9 stops the ultrasonic waves (stops generating ultrasonic waves). The predetermined amount is determined in advance in consideration of appropriate formation of the seal portion S. Also, the predetermined amount is determined such that the generation of ultrasonic waves is stopped before the sealing unit 10 (the anvil 14 and the ultrasonic horn 8) reaches the angular position A3 (in the present embodiment, as shown in FIG. 9, the ultrasonic wave generator 9 stops generating ultrasonic waves at the angular position B2).

When the sealing unit 10 (the anvil 14 and the ultrasonic horn 8) reaches the angular position A3, the anvil 14 and the ultrasonic horn 8 unclamp the continuous web 30 (the anvil 14 moves away from the ultrasonic horn 8, thus unclamping the continuous web). Then, as the sealing unit 10 (the anvil 14 and the ultrasonic horn 8) moves from the angular position A3 to the angular position A0, the follower 56b is guided toward the rotation center line O-O by the cam groove 61, and the anvil 14 rotates away from the ultrasonic horn 8 and the continuous web 30. When the angular position A0 is reached, as shown by the sealing unit 10 at the bottom in FIG. 4 again, the anvil 14 rotates to a retracted position that is at an angle of about 90 degrees relative to the rotation center line O-O.

Note that as shown in FIG. 9, when the sealing unit 10 (the anvil 14 and the ultrasonic horn 8) reaches a predetermined angular position C2 that is along the path from the angular position A3 to the angular position A0, the ultrasonic horn 8 moves from a contact state of being in contact with the continuous web 30 to a separated state of being separated from the continuous web 30. After separating from the ultrasonic horn 8, the continuous web 30 is transported out of the sealing device 1 by the discharge transporting roll 22, and is cut at the cut lines C-C shown in the lower diagram in FIG. 1, thus producing individual pull-on disposable diapers.

### Operation timing in frequency adjusting (tuning) function

It has already be mentioned that the ultrasonic wave generator 9 is provided with a frequency adjusting (tuning) function, and this frequency adjustment is executed during the operation of the sealing device 1. The timing of execution of the frequency adjustment is indicated by an angular position BT in FIG. 9.

Specifically, the frequency of the ultrasonic waves that the ultrasonic wave generator 9 generates for the ultrasonic horn 8 is adjusted (a frequency adjusting step is performed) at a time that is between when the ultrasonic wave generator 9 ends the generation of ultrasonic waves for the ultrasonic horn 8 (see the angular position B2 in FIG. 9) in one instance of processing for forming the seal portions S and when the ultrasonic wave generator 9 starts to generate ultrasonic waves for the ultrasonic horn 8 (see the angular position B1 in FIG. 9) in the next instance of processing for forming the seal portions S by the ultrasonic horn 8.

Also, in one instance of processing for forming the seal portions S, the ultrasonic wave generator 9 ends the generation of ultrasonic waves for the ultrasonic horn 8 (see the angular position B2 in FIG. 9), and thereafter the ultrasonic horn 8 and the anvil 14 unclamp the continuous web 30 (see the angular position A3 in FIG. 9) . Then in the next instance of processing for forming the seal portions S by the ultrasonic horn 8, the ultrasonic horn 8 and the anvil 14 clamp the continuous web 30 (see the angular position A2 in FIG. 9), and then the ultrasonic wave generator 9 starts to generate ultrasonic waves for the ultrasonic horn 8 (see the angular position B1 in FIG. 9). Here, the frequency adjusting step is executed between when the ultrasonic horn 8 and the anvil 14 unclamp the continuous web 30 (see the angular position A3 in FIG. 9) and when the ultrasonic horn 8 and the anvil 14 subsequently clamp the continuous web 30 (see the angular position A2 in FIG. 9).

Also, after the ultrasonic horn 8 and the anvil 14 unclamp the continuous web 30 (see the angular position A3 in FIG. 9), the ultrasonic horn 8 moves from a contact state of being in contact with the continuous web 30 to a separated state of being separated from the continuous web 30 (see the angular position C2 in FIG. 9). Then the ultrasonic horn 8 returns from the separated state to the contact state (see the angular position C1 in FIG. 9), and thereafter the ultrasonic horn 8 and the anvil 14 subsequently clamp the continuous web 30 (see the angular position A2 in FIG. 9). Here, the frequency adjusting step is executed between when the ultrasonic horn 8 moves to the separated state (see the angular position C2 in FIG. 9) and when the ultrasonic horn 8 returns to the contact state (see the angular position C1 in FIG. 9).

In particular, in the present embodiment, when the sealing unit 10 (the anvil 14 and the ultrasonic horn 8) rotates along with rotation of the rotation member and reaches the predetermined angular position BT (corresponding to a predetermined third angular position) that is along the path from the angular position A3 to the angular position A2, a frequency adjusting step is executed. In other words, the frequency adjusting step is executed when the ultrasonic horn 8 and the anvil 14 reach a predetermined angular position. In the present embodiment, an electrical signal is used to notify the ultrasonic wave generator 9 that the sealing unit 10 (the anvil 14 and the ultrasonic horn 8) has reached the angular position BT, and upon receiving that notification, the ultrasonic wave generator 9 adjusts the frequency of the ultrasonic waves that are to be emitted for the ultrasonic horn 8.

Also, in the present embodiment, the frequency adjusting step is executed each time the angular position BT has been reached by the sealing unit 10 N times (N being a natural number of 2 or more) while rotating along with rotation of the rotation member. In other words, instead of the frequency adjusting step being executed every time the sealing unit 10 reaches the angular position BT while rotating along with the rotation of the rotation member, the frequency adjusting step is executed at a rate of once every N times. In this example, N=50, and the frequency adjusting step is performed once every 50 times.

### Operations of six sealing units 10

Whereas the above description of operations is focused on one of the six sealing units 10 provided in the sealing device 1, the following describes how the six sealing units 10 operate, that is to say the relationship between the operations of the six sealing units 10, with reference to FIG. 10. FIG. 10 is an illustrative diagram for illustrating operations of the six sealing units 10.

The six sealing units 10 all perform the previously described operations. In other words, when the sealing units 10 reach the previously described angular positions A0, A1, A2, A3, B1, and B2, they perform the previously described operations that correspond to those angular positions (note that as previously described, the generation of ultrasonic waves is stopped when the ultrasonic vibration energy reaches the predetermined amount, and therefore the ultrasonic waves do not necessarily stop when the angular position B2 is reached. However, in the present embodiment, for the sake of convenience, the following description is based on the presumption that the ultrasonic waves have stopped at the angular position B2 for all of the six sealing units 10).

However, as previously described, the six sealing units 10 are provided at equal intervals corresponding to an arrangement angle of 60 degrees along the circumferential direction of the rotation member that rotates, and therefore there is a time difference between when the sealing units 10 reach each of the angular positions. Accordingly, there is also a difference between the operation timings of the operations of the sealing units 10.

The top section in FIG. 10 shows rotation positions of the rotation member (the rotation base 6 and the rotating drum 5) . In the present embodiment, the rotation positions of the rotation member are considered to be the same as the angular positions of the sealing unit S1, which is one of the six sealing unit 10. In other words, when the rotation member is located at the 0 degree rotation position, the sealing unit S1 is also located at the 0 degree angular position.

The sections below the "rotation position of rotation member" field show operations performed by the sealing unit S1, the sealing unit S2, the sealing unit S3, the sealing unit S4, the sealing unit S5, and the sealing unit S6 in correspondence with the rotation positions. For each sealing unit, these operations are divided into operations of the anvil 14 and operations of the ultrasonic wave generator 9.

As previously described, each sealing unit 10 operates in the sequence of: frequency adjustment (note: once every 50 times) <angular position BT> → start of movement for clamping <angular position A1> → clamping <angular position A2> → ultrasonic wave generation <angular position B1> → stop of ultrasonic waves <angular position B2> → unclamping (start of movement for retraction) <angular position A3> → retraction <angular position A0> → and so on; and as shown in the row for "sealing unit S1", these operations are respectively executed when the rotation position of the rotation member reaches about 20 degrees, about 70 degrees, about 130 degrees, about 140 degrees, about 230 degrees, about 250 degrees, and about 320 degrees. Note that because the rotation positions of the rotation member and the angular positions of the sealing unit S1 match each other, the sealing unit S1 is also located at the angular positions of about 20 degrees, about 70 degrees, about 130 degrees, about 140 degrees, about 230 degrees, about 250 degrees, and about 320 degrees when performing the aforementioned operations.

Also, the sealing unit S2 is the sealing unit 10 that is adjacent to the sealing unit S1 in the circumferential direction, and is separated by an angle of 60 degrees from the sealing unit S1. Accordingly, the sealing unit S2 performs the same operations as the sealing unit S1, at a delay of 60 degrees from the sealing unit S1. Accordingly, the operation graph in the row for "sealing unit S2" corresponds to a 60-degree shift rightward from the operation graph in the row for "sealing unit S1", and the above-described operations are executed when the rotation position of the rotation member reaches angles obtained by adding 60 degrees to the angles for the sealing unit S1, that is to say about 80 degrees, about 130 degrees, about 190 degrees, about 200 degrees, about 290 degrees, about 310 degrees, and about 20 degrees. Note that for these operations, the sealing unit S2 is also located at the angular positions of about 20 degrees, about 70 degrees, about 130 degrees, about 140 degrees, about 230 degrees, about 250 degrees, and about 320 degrees, and this is similar to the case of the sealing unit S1 (i.e., the rotation positions of the rotation member are different for the operations, but the angular positions of the sealing unit 10 are the same for the operations).

Hereinafter, operations are similarly executed in the case of the sealing units S3 to S6 as well. Specifically, the sealing unit S3 is the sealing unit 10 that is adjacent to the sealing unit S2 in the circumferential direction, the sealing unit S4 is the sealing unit 10 that is adjacent to the sealing unit S3 in the circumferential direction, the sealing unit S5 is the sealing unit 10 that is adjacent to the sealing unit S4 in the circumferential direction, and the sealing unit S6 is the sealing unit 10 that is adjacent to the sealing unit S5 in the circumferential direction. Accordingly, the sealing units S3 to S6 perform the same operations as the sealing unit S1 at angles that are respectively delayed by 120 degrees, 180 degrees, 240 degrees, and 300 degrees from the sealing unit S1. Accordingly, the operation graphs in the rows for "sealing unit S3", "sealing unit S4", "sealing unit S5", and "sealing unit S6" are respectively shifted 120 degrees, 180 degrees, 240 degrees, and 300 degrees rightward from the operation graph in the row for "sealing unit S1". Note that the sealing units S3 to S6 are likewise configured such that the above-described operations performed by the sealing unit 10 upon reaching the angular positions of about 70 degrees, about 130 degrees, about 140 degrees, about 230 degrees, about 250 degrees, and about 320 degrees.

In this way, the above-described operations are performed in the sequence of sealing unit S1 → sealing unit S2 → sealing unit S3 → sealing unit S4 → sealing unit S5 → sealing unit S6 → and so on. Accordingly, processing for forming the seal portions S is executed in this sequence, and the seal portions S are formed at intervals in the direction of transporting the continuous web 30. (seal-portion forming step) In other words, the formation of seal portions S with intervals therebetween is sequentially executed as the sealing units 10 each rotate along with rotation of the rotation member.

Note that as previously described, the frequency adjusting step is performed at a rate of once every 50 times (in other words, at a rate of once every time the rotation member makes 50 full rotations) . Accordingly, seal-portion formation processing is normally performed 300 times (=6×50) between when the frequency adjusting step is executed one time and when the frequency adjusting step is executed the next time. In other words, in the present embodiment, the frequency adjusting step is periodically executed such that seal-portion formation processing is executed a predetermined number of times between when the frequency adjusting step is executed one time and when the frequency adjusting step is executed the next time.

### Effectiveness of method for ultrasonic-sealing of present embodiment

As previously described, in the method for ultrasonic-sealing of the present embodiment, the frequency adjusting step for adjusting the frequency of the ultrasonic waves that the ultrasonic wave generator 9 generates for the ultrasonic horn 8 is executed at a time that is between when the ultrasonic wave generator 9 ends the generation of ultrasonic waves for the ultrasonic horn 8 (the angular position B2) in one instance of processing for forming the seal portions S, and when the ultrasonic wave generator 9 starts to generate ultrasonic waves for the ultrasonic horn 8 in the next instance of processing for forming the seal portions S by the ultrasonic horn 8 (the angular position B1). More specifically, the frequency of the ultrasonic waves that the ultrasonic wave generator 9 generates for the ultrasonic horn 8 is adjusted between when the ultrasonic horn 8 and the anvil 14 unclamp the continuous web 30 (the angular position A3) and when the ultrasonic horn 8 and the anvil 14 subsequently clamp the continuous web 30 (the angular position A2). For this reason, it is possible to suppress the problem that the frequency generated by the ultrasonic wave generator 9 ceases to match the natural frequency of the ultrasonic horn 8 when the processing for forming the seal portions S is executed (i.e., it is possible to make the two frequencies match as much as possible).

In conventional examples, the frequency adjusting step is performed when the sealing device 1 is powered on (starts up). However, the following problems have occurred in such cases. Specifically, the ultrasonic horn 8 generates frictional heat in the continuous web 30 while performing the seal-portion formation processing, and therefore the ultrasonic horn 8 stretches/contracts due to the thermal influence of the frictional heat. As this seal-portion formation processing continues for an extended period of time, there has been a possibility that the natural frequency of the ultrasonic horn 8, which changes along with stretching/contracting, will greatly diverge from the ultrasonic frequency that was set by adjustment at power-on (startup).

When this situation occurs, it has been possible for problems such as the following to occur. For example, there have been cases where if the ultrasonic frequency and the natural frequency diverge greatly from each other, an error occurs with the ultrasonic wave generator 9, and the seal-portion formation processing is interrupted.

Also, even in the case where the frequencies do not diverge very much, and the seal-portion formation processing is not interrupted, it has been possible for a sealing defect to occur in the continuous web 30. Specifically, if the ultrasonic frequency and the natural frequency do not match, the ultrasonic wave generator 9 changes the ultrasonic frequency so as to match the natural frequency before starting to generate ultrasonic waves in the seal-portion formation processing. However, the ultrasonic wave generator 9 consumes energy (electric energy) when performing such processing. For this reason, consider the case where, as in the present embodiment, the ultrasonic wave generator 9 starts to generate ultrasonic waves when executing seal-portion formation processing, and then stops generating ultrasonic waves when the ultrasonic vibration energy (electric energy) reaches a predetermined amount, for example. In such a case, the amount of energy consumed by the ultrasonic wave generator 9 for adjusting the ultrasonic frequency cannot be used as energy for seal portion formation, and therefore ultrasonic wave generation will stop even though seal portion formation is incomplete. Accordingly, a sealing defect will occur in the continuous web 30.

In contrast, in the present embodiment, the frequency adjusting step is executed at a time that is between when the ultrasonic wave generator 9 ends the generation of ultrasonic waves for the ultrasonic horn 8 in one instance of processing for forming the seal portions S, and when the ultrasonic wave generator 9 starts to generate ultrasonic waves for the ultrasonic horn 8 in the next instance of processing for forming the seal portions S by the ultrasonic horn 8 (more specifically, between when the ultrasonic horn 8 and the anvil 14 unclamp the continuous web 30 until when the ultrasonic horn 8 and the anvil 14 subsequently clamp the continuous web 30) . For this reason, frequency adjustment is performed while processing for forming the seal portions S continues to be successively executed, and there is a reduced possibility that the natural frequency of the ultrasonic horn 8, which changes according to stretching/contracting, will greatly diverge from the ultrasonic frequency. In other words, it is possible to suppress the problem that the frequency generated by the ultrasonic wave generator 9 ceases to match the natural frequency of the ultrasonic horn 8, and it is possible to suppress the occurrence of the problem of an interruption in the seal-portion formation processing, the problem of a sealing defect, and the like.

Also, in the present embodiment, the frequency of the ultrasonic waves generated by the ultrasonic wave generator 9 is adjusted at a time between when the ultrasonic horn 8 moves to the separated state (the angular position C2) and when the ultrasonic horn 8 returns to the contact state (the angular position C1).

For this reason, frequency adjustment is executed in the so-called naked state in which the ultrasonic horn 8 is in contact with neither the anvil 14 nor the continuous web 30 (is not in contact with anything) . Accordingly, frequency adjustment (tuning) can be performed swiftly and accurately.

Also, in the present embodiment, the frequency adjusting step is executed when the sealing unit 10 reaches the predetermined angular position BT along the path from the predetermined angular position A3 to the predetermined angular position A2 while rotating along with rotation of the rotation member.

In other words, the frequency adjusting step is necessarily executed when the sealing unit 10 reaches a predetermined angular position. For this reason, the frequency adjusting step can be reliably executed at a time that is between when the ultrasonic wave generator 9 ends the generation of ultrasonic waves for the ultrasonic horn 8 in one instance of processing for forming the seal portions S and when the ultrasonic wave generator 9 starts to generate ultrasonic waves for the ultrasonic horn 8 in the next instance of processing for forming the seal portions S by the ultrasonic horn 8.

Also, in the present embodiment, the frequency adjusting step is executed each time the predetermined angular position BT has been reached by the sealing unit 10 N times (N being a natural number of 2 or more) while rotating along with rotation of the rotation member.

As previously described, the objective of the frequency adjusting step in the present embodiment is to suppress the influence that frictional heat generated in the continuous web 30 has on the ultrasonic horn 8. In other words, if frequency adjustment is executed every time the sealing unit 10 reaches the predetermined angular position BT (each time the rotation member makes one full rotation), this can be said to be too often, and results in needless energy consumption. For this reason, in the present embodiment, frequency adjustment is executed each time the rotation member makes two or more full rotations. Frequency adjustment can therefore be performed at an appropriate timing.

### Other embodiments

Although the embodiment of the present disclosure has been described hereinabove, the above embodiment of the present disclosure is simply to facilitate understanding of the present disclosure and are not in any way to be construed as limiting the present disclosure.

Also, in the above embodiment, the frequency adjusting step is periodically executed such that seal-portion formation processing is executed a predetermined number of times between when the frequency adjusting step is executed one time and when the frequency adjusting step is executed the next time. However, there is no limitation to this, and the frequency adjusting step may be periodically executed at a predetermined time interval.

There are cases where multiple seal portions S are formed in a low speed mode in which the continuous web 30 is transported by rotation of the rotation member at a first speed and a high speed mode in which the continuous web 30 is transported by rotation of the rotation member at a second speed that is higher than the first speed. Also, the number of times that the seal-portion formation processing is executed in a predetermined time period is different between the two operation modes, and therefore the extent of the stretching/contracting of the ultrasonic horn 8 in the predetermined time period is also different (the extent of stretching/contracting is higher in the high speed mode), and the extent of divergence between the ultrasonic frequency and the natural frequency is also different.

In the above embodiment, the rate of execution of the frequency adjusting step is set based on the number of times that seal-portion formation processing is performed rather than based on the time from one execution of the frequency adjusting step until the next execution of the frequency adjusting step. Therefore, the frequency adjusting step can be executed at appropriate intervals (before the extent of divergence becomes large) regardless of which mode the operation mode is. The above-described embodiment is desirable in this respect.

Also, in the above embodiment, a different ultrasonic wave generator 9 generates ultrasonic waves for each of the sealing units 10. In other words, one ultrasonic wave generator 9 is provided for each of the sealing units 10, but there is no limitation to this. For example, instead of the ultrasonic wave generators 9 and the sealing units 10 being provided in one-to-one correspondence, one ultrasonic wave generator 9 may be provided in correspondence with multiple sealing units 10.

In the case of providing one ultrasonic wave generator 9 for each of the sealing units 10, one ultrasonic wave generator 9 is not required to perform frequency adjustment for multiple sealing units 10. For this reason, the ultrasonic wave generator 9 does not need to perform reading/writing/switching a memory for each one of multiple corresponding sealing units 10 (each time the corresponding sealing unit 10 changes) . Also, time is not required for such reading/writing/switching, and the ultrasonic wave generator 9 need only perform frequency adjustment for one sealing unit 10, and therefore even if the above-described operation mode is the high speed mode, frequency adjustment can be performed without any problem (with sufficient allowance). The above embodiment is desirable in this respect.

Also, in the above embodiment, when processing for forming a seal portion S is executed, the ultrasonic wave generator 9 starts to generate ultrasonic waves, and then the ultrasonic wave generator 9 stops generating ultrasonic waves when the ultrasonic vibration energy (electric energy) reaches a predetermined amount, but there is no limitation to this. For example, the generation of ultrasonic waves may be stopped after a certain time has elapsed since the ultrasonic wave generator 9 started to generate ultrasonic waves, or when the instantaneous value of the ultrasonic vibration energy of the ultrasonic waves generated by the ultrasonic wave generator 9 (corresponding to the power of the ultrasonic wave generator 9) has exceeded a predetermined threshold value.

Also, in the above embodiment, either the ultrasonic horns 8 or the anvils 14 (first members) are provided on the outer circumferential surface 5A of the rotation member (rotating drum 8), and the other ones (second members) are provided on the opposite side of the continuous web so as to be able to move toward and away from the first members (ultrasonic horns 8) . In the above embodiment, the first members are the ultrasonic horns 8 and the second members are the anvils 14. However, there is no limitation to this. The first members may be the anvils 14, and the second members may be the ultrasonic horns 8.

Also, although the sealing device 1 is provided with six sealing units 10 in the above embodiment, there is no limitation to this. The sealing device 1 may be provided with seven or more sealing units 10, or five or fewer sealing units 10.

Also, although a link mechanism that employs a cam is described as the swing drive portion in the above embodiment, there is no limitation to this. The operations of the ultrasonic horn 8 and the anvil 14 in the above embodiment may be realized with use of a cylinder mechanism or the like instead of a link mechanism that employs a cam.

Also, although an air damper is used as the first elastic member 71 and the second elastic member 72 in the above embodiment, there is no limitation to this. A coil spring or the like may be used instead of an air damper.

Also, the frequency adjusting step described below may be performed in addition to the frequency adjusting step of the above embodiment.

First, if the repeatedly executed processing for forming the seal portions S is paused, the frequency of the ultrasonic waves generated by the ultrasonic wave generator 9 may be adjusted when processing is resumed. For example, a configuration is possible in which if the processing for forming the seal portions S (or the overall absorbent article continuous production line) is paused due to some sort of circumstance, the pause is detected and recorded, and frequency adjustment is always executed when processing is resumed, regardless of how much time elapses before resumption.

Also, in the above description, it is described that when processing for forming the seal portions S is executed, the seal portions S are formed while the frequency of the ultrasonic waves generated by the ultrasonic wave generator 9 is adjusted (while the ultrasonic frequency is changed so as to follow change in the natural frequency), but a configuration is possible in which the final frequency when the ultrasonic wave generator 9 stops generating ultrasonic waves is stored in a storage unit, and then when processing for forming the seal portions S is to be performed next, the ultrasonic wave generator 9 starts to generate ultrasonic waves at the frequency stored in the storage unit.

Using these frequency adjusting steps together makes it possible to further suppress the problem that the frequency generated by the ultrasonic wave generator 9 ceases to match the natural frequency of the ultrasonic horn 8.

### [Reference Signs List]

1 sealing device, 2 timing wheel, 3 bearing portion, 3a rotation shaft 3b ball bearing, 4 fixed table, 5 rotating drum, 5A outer circumferential surface
5a rotating drum window, 6 rotation base, 7a converter, 7b booster 8 ultrasonic horn, 8a horn facing surface, 9 ultrasonic wave generator, 10 sealing unit
14 anvil, 14B anvil projection portion, 14a anvil facing surface
21 supply transporting roll, 22 discharge transporting roll, 30 continuous web
30A one side of continuous web, 30B other side of continuous web, 31 second sheet
32 first sheet, 32a side edge on one side of first sheet
32b side edge on other side of first sheet, 33 liquid-absorbent body, 34 leg hole
35 first waist band, 36 second waist band, 37 first leg band
38 second leg band, 50 swinging support member, 51 swing portion, 51A swing shaft
51B first coupling shaft, 52 holding portion, 52B attachment surface, 52a frame portion, 52c inward surface
53 drive link, 54 rotation link, 54A rotation link base portion
54B rotation link arm portion, 54a support shaft, 55 second coupling shaft, 56 drive member
56a drive support body, 56b follower, 60 cam member, 61 cam groove
70 support portion, 71 first elastic member, 71A first fixing plate, 71B second fixing plate
72 second elastic member, 73 first nozzle, 74 second nozzle, 75 first air pipe
76 second air pipe, S seal portion

## Claims

1. A method for ultrasonic-sealing in which a seal portion (S) is formed in a continuous web (30) for an absorbent article,
the forming being performed by vibration of the ultrasonic horn (8) due to receiving an ultrasonic wave generated by an ultrasonic wave generator (9) while the continuous web (30) is being transported and clamped between an ultrasonic horn (8) and an anvil (14),
the method for ultrasonic-sealing comprising:
a seal-portion forming step of forming a plurality of the seal portions at an interval in a direction of transporting the continuous web (30) by repeatedly executing processing for forming the seal portion (S); and **characterized by**
a frequency adjusting step of adjusting a frequency of the ultrasonic wave generated by the ultrasonic wave generator (9) for the ultrasonic horn (8) and storing the adjusted frequency in a storage unit,
the adjusting and the storing being performed at a time between
when the ultrasonic wave generator (9) ends the generation of the ultrasonic wave for the ultrasonic horn (8) in one instance of processing for forming the seal portion (S) and
when the ultrasonic wave generator (9) starts to generate the ultrasonic wave for the ultrasonic horn (8) in a subsequent instance of processing for forming the seal portion (S) by the ultrasonic horn (8),
the ultrasonic wave generator (9) starting to generate the ultrasonic wave at the frequency stored in the storage unit when the processing for forming the seal portion (S) is to be executed.

2. The method for ultrasonic-sealing according to claim 1, wherein
when the processing for forming the seal portion (S) is to be executed,
the seal portion is formed while following the frequency of the ultrasonic wave generated by the ultrasonic wave generator (9), and
the frequency at a time when the ultrasonic wave generator (9) ends the generation of the ultrasonic wave is stored in the storage unit.

3. The method for ultrasonic-sealing according to claim 1 or 2, wherein
in the processing for forming the seal portion (S),
the ultrasonic horn (8) and the anvil (14) unclamp the continuous web (30) after the ultrasonic wave generator (9) has stopped generating the ultrasonic wave for the ultrasonic horn (8),
in the subsequent processing for forming the seal portion (S) by the ultrasonic horn (8),
the ultrasonic wave generator (9) starts to generate the ultrasonic wave for the ultrasonic horn (8) after the ultrasonic horn (8) and the anvil (14) have clamped the continuous web (30), and
in the frequency adjusting step,
the frequency of the ultrasonic wave generated by the ultrasonic wave generator (9) for the ultrasonic horn (8) is adjusted at a time between when the ultrasonic horn (8) and the anvil (14) unclamp the continuous web (30) and when the ultrasonic horn (8) and the anvil (14) subsequently clamp the continuous web (30).

4. The method for ultrasonic-sealing according to claim 3, wherein
after the ultrasonic horn (8) and the anvil (14) unclamp the continuous web (30),
the ultrasonic horn (8) moves from a contact state of being in contact with the continuous web (30) to a separated state of being separated from the continuous web (30),
after the ultrasonic horn (8) has returned from the separated state to the contact state,
the ultrasonic horn (8) and the anvil (14) subsequently clamp the continuous web (30), and
in the frequency adjusting step,
the frequency of the ultrasonic wave generated by the ultrasonic wave generator (9) is adjusted at a time between when the ultrasonic horn (8) moves to the separated state and when the ultrasonic horn (8) returns to the contact state.

5. The method for ultrasonic-sealing according to any of claims 1 to 4, wherein
in the frequency adjusting step,
the ultrasonic wave generator (9) generates the ultrasonic wave, adjusting the frequency of the ultrasonic wave, and
an amplitude of the ultrasonic wave generated by the ultrasonic wave generator (9) in the frequency adjusting step is smaller than an amplitude of the ultrasonic wave generated by the ultrasonic wave generator (9) in the seal-portion forming step.

6. The method for ultrasonic-sealing according to any of claims 1 to 5, wherein
the frequency adjusting step is periodically executed such that the processing for forming the seal portion (S) is performed a predetermined number of times between when the frequency adjusting step is executed and when the frequency adjusting step is subsequently executed.

7. The method for ultrasonic-sealing according to any of claims 1 to 6, wherein
the continuous web (30) is transported by rotation of the rotation member with being wound on an outer circumferential surface of a rotation member,
a plurality of sealing units that each have an ultrasonic horn (8) and an anvil (14) are provided at an equal spacing along a circumferential direction of the rotation member,
a plurality of first members, which are either of the ultrasonic horns (8) and the anvils (14), are provided on the outer circumferential surface of the rotation member,
a plurality of second members, which are other ones out of the ultrasonic horns and the anvils, are each provided on an opposite side of the continuous web (30) so as to be capable of moving toward and away from the corresponding first member,
for each of the sealing units, when the sealing unit reaches a predetermined first angular position while rotating along with rotation of the rotation member,
the second member comes into contact with the corresponding first member and clamps the continuous web (30),
for each of the sealing units, when the sealing unit reaches a predetermined second angular position while rotating along with rotation of the rotation member,
the second member moves away from the first member and unclamps the continuous web (30), and
for each of the sealing units, when the sealing unit reaches a predetermined third angular position along a path from the predetermined second angular position to the predetermined first angular position while rotating along with rotation of the rotation member, the sealing unit execute the frequency adjusting step.

8. The method for ultrasonic-sealing according to claim 7, wherein
for each of the sealing units, the frequency adjusting step is executed each time the predetermined third angular position has been reached by the sealing unit N times, N being a natural number of 2 or more, while rotating along with rotation of the rotation member.

9. The method for ultrasonic-sealing according to any of claims 1 to 8, wherein
the continuous web (30) is transported by rotation of the rotation member with being wound on an outer circumferential surface of a rotation member,
a plurality of sealing units that each have an ultrasonic horn and an anvil are provided at an equal spacing along a circumferential direction of the rotation member,
a plurality of first members, which are either of the ultrasonic horns and the anvils, are provided on the outer circumferential surface of the rotation member,
a plurality of second members, which are other ones out of the ultrasonic horns (8) and the anvils (14), are each provided on an opposite side of the continuous web (30) so as to be capable of moving toward and away from the corresponding first member,
the plurality of sealing units sequentially form the plurality of seal portions at the interval while rotating along with rotation of the rotation member, and
a different ultrasonic wave generator (9) generates the ultrasonic wave for each of the sealing units.

10. The method for ultrasonic-sealing according to any of claims 1 to 9, wherein
when the processing for forming the seal portion (S) is executed,
the ultrasonic wave generator (9) starts to generate the ultrasonic wave, and then the ultrasonic wave generator (9) stops generating the ultrasonic wave when an ultrasonic vibration energy has reached a predetermined amount.

11. The method for ultrasonic-sealing according to any of claims 1 to 10, wherein
in a case where the repeatedly executed processing for forming the seal portion (S) is paused,
the frequency of the ultrasonic wave generated by the ultrasonic wave generator (9) is adjusted when processing is resumed.

12. A device for ultrasonic-sealing that forms a plurality of seal portions in a continuous web (30) that is for an absorbent article,
the device for ultrasonic-sealing comprising:
a transporting device configured to transport the continuous web (30);
an ultrasonic wave generator (9) configured to generate an ultrasonic wave;
an ultrasonic horn configured to form the seal portion (S) in the continuous web (30) that is being transported by the transporting device, by vibrating due to receiving the ultrasonic wave generated by the ultrasonic wave generator (9); and
an anvil (14) configured to clamp the continuous web (30) together with the ultrasonic horn (8),
the ultrasonic horn (8) being configured to form the plurality of seal portions at an interval in a direction of transporting the continuous web (30) by repeatedly executing ultrasonic-sealing processing,
the ultrasonic-sealing processing being configured to form a seal portion in the continuous web (30) by vibrating while clamping the continuous web (30) together with the anvil (14), the device being **characterized by**
the ultrasonic wave generator (9) being configured to execute a frequency adjusting step of adjusting a frequency of the ultrasonic wave generated by the ultrasonic wave generator (9) for the ultrasonic horn (8), at a time between
when the ultrasonic wave generator (9) ends the generation of the ultrasonic wave for the ultrasonic horn (8) in one instance of processing for forming the seal portion (S) and
when the ultrasonic wave generator (9) starts to generate the ultrasonic wave for the ultrasonic horn (8) in a subsequent instance of processing for forming the seal portion (S) by the ultrasonic horn (8).

## Patentansprüche

1. Verfahren zum Ultraschallsiegeln, wobei ein Siegelabschnitt (S) in einer kontinuierlichen Bahn (30) für einen absorbierenden Artikel gebildet wird,
wobei das Bilden durch Vibration des Ultraschallhorns (8) aufgrund des Empfangens einer Ultraschallwelle durchgeführt wird, die durch einen Ultraschallwellengenerator (9) erzeugt wird, während die kontinuierliche Bahn (30) transportiert und zwischen einem Ultraschallhorn (8) und einem Amboss (14) eingeklemmt wird,
wobei das Verfahren zum Ultraschallsiegeln Folgendes umfasst:
einen Siegelabschnittsbildungsschritt zum Bilden einer Vielzahl von Siegelabschnitten in einem Intervall in einer Richtung des Transportierens der kontinuierlichen Bahn (30) durch wiederholtes Ausführen einer Verarbeitung zum Bilden des Siegelabschnitts (S); und **gekennzeichnet durch**
einen Frequenzeinstellungsschritt zum Einstellen einer Frequenz der Ultraschallwelle, die durch den Ultraschallwellengenerator (9) erzeugt wird, für das Ultraschallhorn (8) und Speichern der eingestellten Frequenz in einer Speichereinheit,
wobei das Einstellen und Speichern zu einem Zeitpunkt durchgeführt wird zwischen
einem Ende der Erzeugung der Ultraschallwelle durch den Ultraschallwellengenerator (9) für das Ultraschallhorn (8) in einer Instanz der Verarbeitung zum Bilden des Siegelabschnitts (S) und
einem Start der Erzeugung der Ultraschallwelle durch den Ultraschallwellengenerator (9) für das Ultraschallhorn (8) in einer nachfolgenden Instanz der Verarbeitung zum Bilden des Siegelabschnitts (S) durch das Ultraschallhorn (8),
wobei der Ultraschallwellengenerator (9) damit startet, die Ultraschallwelle in der Frequenz, die in der Speichereinheit gespeichert ist, zu erzeugen, wenn die Verarbeitung zum Bilden des Siegelabschnitts (S) auszuführen ist.

2. Verfahren zum Ultraschallsiegeln nach Anspruch 1, wobei
wenn die Verarbeitung zum Bilden des Siegelabschnitts (S) auszuführen ist,
der Siegelabschnitt gebildet wird, während der Frequenz der Ultraschallwelle gefolgt wird, die durch den Ultraschallwellengenerator (9) erzeugt wird, und
die Frequenz zu einem Zeitpunkt, zu dem der Ultraschallwellengenerator (9) die Erzeugung der Ultraschallwelle beendet, in der Speichereinheit gespeichert wird.

3. Verfahren zum Ultraschallsiegeln nach Anspruch 1 oder 2, wobei
bei der Verarbeitung zum Bilden des Siegelabschnitts (S),
das Ultraschallhorn (8) und der Amboss (14) die kontinuierliche Bahn (30) freigeben, nachdem der Ultraschallwellengenerator (9) das Erzeugen der Ultraschallwelle für das Ultraschallhorn (8) gestoppt hat,
bei der nachfolgenden Verarbeitung zum Bilden des Siegelabschnitts (S) durch das Ultraschallhorn (8),
der Ultraschallwellengenerator (9) damit startet, die Ultraschallwelle für das Ultraschallhorn (8) zu erzeugen, nachdem das Ultraschallhorn (8) und der Amboss (14) die kontinuierliche Bahn (30) eingeklemmt haben, und
in dem Frequenzeinstellungsschritt,
die Frequenz der Ultraschallwelle, die durch den Ultraschallwellengenerator (9) für das Ultraschallhorn (8) erzeugt wird, zu einem Zeitpunkt zwischen dem Freigeben der kontinuierlichen Bahn (30) durch das Ultraschallhorn (8) und den Amboss (14) und dem nachfolgenden Einklemmen der kontinuierlichen Bahn (30) durch das Ultraschallhorn (8) und den Amboss (14) eingestellt wird.

4. Verfahren zum Ultraschallsiegeln nach Anspruch 3, wobei
nachdem das Ultraschallhorn (8) und der Amboss (14) die kontinuierlichen Bahn (30) freigegeben haben, sich das Ultraschallhorn (8) aus einem Kontaktzustand, in dem es mit der kontinuierlichen Bahn (30) in Kontakt ist, in einen getrennten Zustand bewegt, in dem es von der kontinuierlichen Bahn (30) getrennt ist,
nachdem das Ultraschallhorn (8) aus dem getrennten Zustand in den Kontaktzustand zurückgekehrt ist,
das Ultraschallhorn (8) und der Amboss (14) die kontinuierliche Bahn (30) nachfolgend einklemmen, und
in dem Frequenzeinstellungsschritt,
die Frequenz der Ultraschallwelle, die durch den Ultraschallwellengenerator (9) erzeugt wird, zu einem Zeitpunkt zwischen dem Bewegen des Ultraschallhorns (8) in den getrennten Zustand und dem Zurückkehren des Ultraschallhorns (8) in den Kontaktzustand eingestellt wird.

5. Verfahren zum Ultraschallsiegeln nach einem der Ansprüche 1 bis 4, wobei
in dem Frequenzeinstellungsschritt,
der Ultraschallwellengenerator (9) die Ultraschallwelle erzeugt, wobei die Frequenz der Ultraschallwelle eingestellt wird, und
eine Amplitude der Ultraschallwelle, die durch den Ultraschallwellengenerator (9) in dem Frequenzeinstellungsschritt erzeugt wird, kleiner ist als eine Amplitude der Ultraschallwelle, die durch den Ultraschallwellengenerator (9) in dem Siegelabschnittsbildungsschritt erzeugt wird.

6. Verfahren zum Ultraschallsiegeln nach einem der Ansprüche 1 bis 5, wobei
der Frequenzeinstellungsschritt periodisch ausgeführt wird, sodass die Verarbeitung zum Bilden des Siegelabschnitts (S) eine vorbestimmte Anzahl von Malen zwischen dem Ausführen des Frequenzeinstellungsschritts und dem nachfolgenden Ausführen des Frequenzeinstellungsschritts durchgeführt wird.

7. Verfahren zum Ultraschallsiegeln nach einem der Ansprüche 1 bis 6, wobei
die kontinuierliche Bahn (30) durch Rotation des Rotationselements transportiert wird, wobei sie auf eine Außenumfangsfläche eines Rotationselements gewickelt wird,
eine Vielzahl von Siegeleinheiten, die jeweils ein Ultraschallhorn (8) und einen Amboss (14) aufweisen, in einem gleichen Abstand entlang einer Umfangsrichtung des Rotationselements bereitgestellt ist,
eine Vielzahl von ersten Elementen, bei der es sich entweder um die Ultraschallhörner (8) oder die Ambosse (14) handelt, an der Außenumfangsfläche des Rotationselements bereitgestellt ist,
eine Vielzahl von zweiten Elementen, bei der es sich um andere von den Ultraschallhörnern und den Ambossen handelt, jeweils auf einer gegenüberliegenden Seite der kontinuierlichen Bahn (30) bereitgestellt ist, um dazu in der Lage zu sein, sich zu dem entsprechenden ersten Element hin und weg davon zu bewegen,
für jede der Siegeleinheiten, wenn die Siegeleinheit während des Rotierens zusammen mit der Rotation des Rotationselements eine vorbestimmte erste Winkelposition erreicht,
das zweite Element mit dem entsprechenden ersten Element in Kontakt kommt und die kontinuierliche Bahn (30) einklemmt,
für jede der Siegeleinheiten, wenn die Siegeleinheit während des Rotierens zusammen mit der Rotation des Rotationselements eine vorbestimmte zweite Winkelposition erreicht,
sich das zweite Element weg von dem ersten Element bewegt und die kontinuierliche Bahn (30) freigibt, und
für jede der Siegeleinheiten, wenn die Siegeleinheit während des Rotierens zusammen mit der Rotation des Rotationselements eine vorbestimmte dritte Winkelposition entlang eines Wegs von der vorbestimmten zweiten Winkelposition zu der vorbestimmten ersten Winkelposition erreicht, die Siegeleinheit den Frequenzeinstellungsschritt ausführt.

8. Verfahren zum Ultraschallsiegeln nach Anspruch 7, wobei
für jede der Siegeleinheiten der Frequenzeinstellungsschritt jedes Mal ausgeführt wird, wenn die vorbestimmte dritte Winkelposition durch die Siegeleinheit N-mal, wobei N eine natürliche Zahl von 2 oder mehr ist, während des Rotierens zusammen mit der Rotation des Rotationselements erreicht wurde.

9. Verfahren zum Ultraschallsiegeln nach einem der Ansprüche 1 bis 8, wobei
die kontinuierliche Bahn (30) durch Rotation des Rotationselements transportiert wird, wobei sie auf eine Außenumfangsfläche eines Rotationselements gewickelt wird,
eine Vielzahl von Siegeleinheiten, die jeweils ein Ultraschallhorn und einen Amboss aufweisen, in einem gleichen Abstand entlang einer Umfangsrichtung des Rotationselements bereitgestellt ist,
eine Vielzahl von ersten Elementen, bei der es sich entweder um die Ultraschallhörner oder die Ambosse handelt, an der Außenumfangsfläche des Rotationselements bereitgestellt ist,
eine Vielzahl von zweiten Elementen, bei der es sich um andere von den Ultraschallhörnern (8) und den Ambossen (14) handelt, jeweils auf einer gegenüberliegenden Seite der kontinuierlichen Bahn (30) bereitgestellt ist, um dazu in der Lage zu sein, sich zu dem entsprechenden ersten Element hin und weg davon zu bewegen,
die Vielzahl von Siegeleinheiten die Vielzahl von Siegelabschnitten in dem Intervall während des Rotierens zusammen mit der Rotation des Rotationselements sequenziell bildet, und
ein anderer Ultraschallwellengenerator (9) die Ultraschallwelle für jede der Siegeleinheiten erzeugt.

10. Verfahren zum Ultraschallsiegeln nach einem der Ansprüche 1 bis 9, wobei
wenn die Verarbeitung zum Bilden des Siegelabschnitts (S) ausgeführt wird,
der Ultraschallwellengenerator (9) damit startet, die Ultraschallwelle zu erzeugen, und der Ultraschallwellengenerator (9) das Erzeugen der Ultraschallwelle dann stoppt, wenn eine Ultraschallvibrationsenergie eine vorbestimmte Menge erreicht hat.

11. Verfahren zum Ultraschallsiegeln nach einem der Ansprüche 1 bis 10, wobei
in einem Fall, in dem die wiederholt ausgeführte Verarbeitung zum Bilden des Siegelabschnitts (S) angehalten ist,
die Frequenz der Ultraschallwelle, die durch den Ultraschallwellengenerator (9) erzeugt wird, eingestellt wird, wenn die Verarbeitung wiederaufgenommen wird.

12. Vorrichtung zum Ultraschallsiegeln, die eine Vielzahl von Siegelabschnitten in einer kontinuierlichen Bahn (30) bildet, die für einen absorbierenden Artikel ist,
wobei die Vorrichtung zum Ultraschallsiegeln Folgendes umfasst:
eine Transportvorrichtung, die dazu konfiguriert ist, die kontinuierliche Bahn (30) zu transportieren;
einen Ultraschallwellengenerator (9), der dazu konfiguriert ist, eine Ultraschallwelle zu erzeugen;
ein Ultraschallhorn, das dazu konfiguriert ist, den Siegelabschnitt (S) in der kontinuierlichen Bahn (30), die durch die Transportvorrichtung transportiert wird, durch Vibrieren aufgrund des Empfangens der Ultraschallwelle, die durch den Ultraschallwellengenerator (9) erzeugt wird, zu bilden; und
einen Amboss (14), der dazu konfiguriert ist, die kontinuierliche Bahn (30) zusammen mit dem Ultraschallhorn (8) einzuklemmen,
wobei das Ultraschallhorn (8) dazu konfiguriert ist, die Vielzahl von Siegelabschnitten in einem Intervall in einer Richtung des Transportierens der kontinuierlichen Bahn (30) durch wiederholtes Ausführen einer Ultraschallsiegelverarbeitung zu bilden,
wobei die Ultraschallsiegelverarbeitung dazu konfiguriert ist, einen Siegelabschnitt in der kontinuierlichen Bahn (30) durch Vibrieren zu bilden, während die kontinuierliche Bahn (30) zusammen mit dem Amboss (14) eingeklemmt ist, wobei die Vorrichtung **dadurch gekennzeichnet ist, dass**
der Ultraschallwellengenerator (9) dazu konfiguriert ist, einen Frequenzeinstellungsschritt zum Einstellen einer Frequenz der Ultraschallwelle, die durch den Ultraschallwellengenerator (9) erzeugt wird, für das Ultraschallhorn (8) zu einem Zeitpunkt auszuführen zwischen
einem Ende der Erzeugung der Ultraschallwelle durch den Ultraschallwellengenerator (9) für das Ultraschallhorn (8) in einer Instanz der Verarbeitung zum Bilden des Siegelabschnitts (S) und
einem Start der Erzeugung der Ultraschallwelle durch den Ultraschallwellengenerator (9) für das Ultraschallhorn (8) in einer nachfolgenden Instanz der Verarbeitung zum Bilden des Siegelabschnitts (S) durch das Ultraschallhorn (8).

## Revendications

1. Procédé de scellement par ultrasons dans lequel une partie de scellement (S) est formée dans une bande continue (30) pour un article absorbant,
la formation étant effectuée par vibration du cornet à ultrasons (8) en raison de la réception d'une onde ultrasonore générée par un générateur d'ondes ultrasonores (9) pendant que la bande continue (30) est transportée et serrée entre un cornet à ultrasons (8) et une enclume (14),
le procédé de scellement par ultrasons comprenant :
une étape de formation de partie de scellement consistant à former une pluralité des parties de scellement à un intervalle dans une direction de transport de la bande continue (30) en exécutant de manière répétée un traitement pour former la partie de scellement (S) ; et **caractérisé par**
une étape de réglage de fréquence consistant à régler une fréquence de l'onde ultrasonore générée par le générateur d'ondes ultrasonores (9) pour le cornet à ultrasons (8) et à stocker la fréquence réglée dans une unité de stockage,
le réglage et le stockage étant exécutés à un instant entre
le moment où le générateur d'ondes ultrasonores (9) termine la génération de l'onde ultrasonore pour le cornet à ultrasons (8) à une instance de traitement pour former la partie de scellement (S) et
le moment où le générateur d'ondes ultrasonores (9) commence à générer l'onde ultrasonore pour le cornet à ultrasons (8) à une instance ultérieure de traitement pour former la partie de scellement (S) par le cornet à ultrasons (8),
le générateur d'ondes ultrasonores (9) commençant à générer l'onde ultrasonore à la fréquence stockée dans l'unité de stockage lorsque le traitement pour former la partie de scellement (S) doit être exécuté.

2. Procédé de scellement par ultrasons selon la revendication 1, dans lequel
lorsque le traitement pour former la partie de scellement (S) doit être exécuté,
la partie de scellement est formée tout en suivant la fréquence de l'onde ultrasonore générée par le générateur d'ondes ultrasonores (9), et
la fréquence à un moment où le générateur d'ondes ultrasonores (9) termine la génération de l'onde ultrasonore est stockée dans l'unité de stockage.

3. Procédé de scellement par ultrasons selon la revendication 1 ou 2, dans lequel
dans le traitement pour former la partie de scellement (S),
le cornet à ultrasons (8) et l'enclume (14) desserrent la bande continue (30) après que le générateur d'ondes ultrasonores (9) a cessé de générer l'onde ultrasonore pour le cornet à ultrasons (8),
dans le traitement ultérieur pour former la partie de scellement (S) par le cornet à ultrasons (8),
le générateur d'ondes ultrasonores (9) commence à générer l'onde ultrasonore pour le cornet à ultrasons (8) après que le cornet à ultrasons (8) et l'enclume (14) ont serré la bande continue (30), et
dans l'étape de réglage de fréquence,
la fréquence de l'onde ultrasonore générée par le générateur d'ondes ultrasonores (9) pour le cornet à ultrasons (8) est réglée à un instant entre le moment où le cornet à ultrasons (8) et l'enclume (14) desserrent la bande continue (30) et le moment où le cornet à ultrasons (8) et l'enclume (14) serrent ensuite la bande continue (30).

4. Procédé de scellement par ultrasons selon la revendication 3, dans lequel
après que le cornet à ultrasons (8) et l'enclume (14) ont desserré la bande continue (30),
le cornet à ultrasons (8) passe d'un état de contact dans lequel il est en contact avec la bande continue (30) à un état séparé dans lequel il est séparé de la bande continue (30),
après que le cornet à ultrasons (8) est revenu de l'état séparé à l'état de contact,
le cornet à ultrasons (8) et l'enclume (14) serrent ensuite la bande continue (30), et
dans l'étape de réglage de fréquence,
la fréquence de l'onde ultrasonore générée par le générateur d'ondes ultrasonores (9) est réglée à un instant entre le moment où le cornet à ultrasons (8) passe à l'état séparé et le moment où le cornet à ultrasons (8) revient à l'état de contact.

5. Procédé de scellement par ultrasons selon l'une quelconque des revendications 1 à 4, dans lequel
dans l'étape de réglage de fréquence,
le générateur d'ondes ultrasonores (9) génère l'onde ultrasonore, en réglant la fréquence de l'onde ultrasonore, et
une amplitude de l'onde ultrasonore générée par le générateur d'ondes ultrasonores (9) à l'étape de réglage de fréquence est inférieure à une amplitude de l'onde ultrasonore générée par le générateur d'ondes ultrasonores (9) à l'étape de formation de la partie de scellement.

6. Procédé de scellement par ultrasons selon l'une quelconque des revendications 1 à 5, dans lequel
l'étape de réglage de fréquence est exécutée périodiquement de sorte que le traitement pour former la partie de scellement (S) est exécuté un nombre prédéterminé de fois entre le moment où l'étape de réglage de fréquence est exécutée et le moment où l'étape de réglage de fréquence est ensuite exécutée.

7. Procédé de scellement par ultrasons selon l'une quelconque des revendications 1 à 6, dans lequel
la bande continue (30) est transportée par rotation de l'élément de rotation en étant enroulée sur une surface circonférentielle externe d'un élément de rotation,
une pluralité d'unités de scellement qui ont chacune un cornet à ultrasons (8) et une enclume (14) sont prévues à un espacement égal le long d'une direction circonférentielle de l'élément de rotation,
une pluralité de premiers éléments, qui sont les unes parmi les cornes à ultrasons (8) et les enclumes (14), sont prévus sur la surface circonférentielle externe de l'élément de rotation,
une pluralité de seconds éléments, qui sont les autres parmi les cornes à ultrasons et les enclumes, sont chacun prévus sur un côté opposé de la bande continue (30) de manière à pouvoir se rapprocher et s'éloigner du premier élément correspondant,
pour chacune des unités de scellement, lorsque l'unité de scellement atteint une première position angulaire prédéterminée tout en tournant avec la rotation de l'élément de rotation,
le second élément vient en contact avec le premier élément correspondant et serre la bande continue (30),
pour chacune des unités de scellement, lorsque l'unité de scellement atteint une deuxième position angulaire prédéterminée tout en tournant avec la rotation de l'élément de rotation,
le second élément s'éloigne du premier élément et desserre la bande continue (30), et
pour chacune des unités de scellement, lorsque l'unité de scellement atteint une troisième position angulaire prédéterminée le long d'un trajet allant de la deuxième position angulaire prédéterminée à la première position angulaire prédéterminée tout en tournant avec la rotation de l'élément de rotation, l'unité de scellement exécute l'étape de réglage de fréquence.

8. Procédé de scellement par ultrasons selon la revendication 7, dans lequel
pour chacune des unités de scellement, l'étape de réglage de fréquence est exécutée chaque fois que la troisième position angulaire prédéterminée a été atteinte par l'unité de scellement N fois, N étant un nombre entier naturel de 2 ou plus, tout en tournant avec la rotation de l'élément de rotation.

9. Procédé de scellement par ultrasons selon l'une quelconque des revendications 1 à 8, dans lequel
la bande continue (30) est transportée par rotation de l'élément de rotation en étant enroulée sur une surface circonférentielle externe d'un élément de rotation,
une pluralité d'unités de scellement qui ont chacune un cornet à ultrasons et une enclume sont prévues à un espacement égal le long d'une direction circonférentielle de l'élément de rotation,
une pluralité de premiers éléments, qui sont les unes parmi les cornes à ultrasons et les enclumes, sont prévus sur la surface circonférentielle externe de l'élément de rotation,
une pluralité de seconds éléments, qui sont les autres parmi les cornes à ultrasons (8) et les enclumes (14), sont chacun prévus sur un côté opposé de la bande continue (30) de manière à pouvoir se rapprocher et s'éloigner du premier élément correspondant,
la pluralité d'unités de scellement forment séquentiellement la pluralité de parties de scellement à l'intervalle tout en tournant avec la rotation de l'élément de rotation, et
un générateur d'ondes ultrasonores différent (9) génère l'onde ultrasonore pour chacune des unités de scellement.

10. Procédé de scellement par ultrasons selon l'une quelconque des revendications 1 à 9, dans lequel
lorsque le traitement pour former la partie de scellement (S) est exécuté,
le générateur d'ondes ultrasonores (9) commence à générer l'onde ultrasonore, puis le générateur d'ondes ultrasonores (9) arrête de générer l'onde ultrasonore lorsqu'une énergie de vibration ultrasonore a atteint une quantité prédéterminée.

11. Procédé de scellement par ultrasons selon l'une quelconque des revendications 1 à 10, dans lequel
dans un cas où le traitement exécuté de manière répétée pour former la partie de scellement (S) est mis en pause,
la fréquence de l'onde ultrasonore générée par le générateur d'ondes ultrasonores (9) est réglée lors de la reprise du traitement.

12. Dispositif de scellement par ultrasons qui forme une pluralité de parties de scellement dans une bande continue (30) qui est destinée à un article absorbant,
le dispositif de scellement par ultrasons comprenant :
un dispositif de transport configuré pour transporter la bande continue (30) ;
un générateur d'ondes ultrasonores (9) configuré pour générer une onde ultrasonore ;
un cornet à ultrasons configuré pour former la partie de scellement (S) dans la bande continue (30) qui est transportée par le dispositif de transport, en vibrant en raison de la réception de l'onde ultrasonore générée par le générateur d'ondes ultrasonores (9) ; et
une enclume (14) configurée pour serrer la bande continue (30) avec le cornet à ultrasons (8),
le cornet à ultrasons (8) étant configuré pour former la pluralité de parties de scellement à un intervalle dans une direction de transport de la bande continue (30) en exécutant de manière répétée un traitement de scellement par ultrasons,
le traitement de scellement par ultrasons étant configuré pour former une partie de scellement dans la bande continue (30) par vibration tout en serrant la bande continue (30) avec l'enclume (14), le dispositif étant **caractérisé par**
le générateur d'ondes ultrasonores (9) étant configuré pour exécuter une étape de réglage de fréquence consistant à régler une fréquence de l'onde ultrasonore générée par le générateur d'ondes ultrasonores (9) pour le cornet à ultrasons (8), à un instant entre
le moment où le générateur d'ondes ultrasonores (9) termine la génération de l'onde ultrasonore pour le cornet à ultrasons (8) à une instance de traitement pour former la partie de scellement (S) et
le moment où le générateur d'ondes ultrasonores (9) commence à générer l'onde ultrasonore pour le cornet à ultrasons (8) à une instance ultérieure de traitement pour former la partie de scellement (S) par le cornet à ultrasons (8).
